# EUROPEAN PATENT APPLICATION

(11) **EP 4 545 716 A1**
(43) Date of publication of application: **30.04.2025**
(21) Application number: 23826456.8
(22) Date of filing: 20.06.2023
(51) Int. Cl.: E03D 11/02, G01N 33/50, G01N 35/10

(54) **TEST PIECE FOR URINALYSIS, TRANSMISSION MECHANISM, AND TOILET**

(30) Priority: 21.06.2022 CN 202221559738 U; 21.06.2022 CN 202221561591 U; 17.08.2022 CN 202222171089 U; 24.11.2022 CN 202223134007 U; 20.12.2022 CN 202223422272 U; 20.12.2022 CN 202223422176 U; 30.01.2023 CN 202320086317 U; 09.03.2023 CN 202320439053 U; 31.03.2023 CN 202320679913 U
(71) Applicant: XIAMEN R&T PLUMBING TECHNOLOGY CO., LTD., Xiamen, Fujian 361028 (CN)
(72) Inventor: LIN, Jian, Xiamen, Fujian 361028 (CN); HUANG, Candong, Xiamen, Fujian 361028 (CN); ZHANG, Zulian, Xiamen, Fujian 361028 (CN); LIU, Zhipeng, Xiamen, Fujian 361028 (CN); ZHONG, Zhijun, Xiamen, Fujian 361028 (CN)
(74) Representative: Loo, Chi Ching
(86) International application number: PCT/CN2023/101499
(87) International publication number: WO 2023/246809

(57) **Abstract**

Disclosed are a test piece for urinalysis, a transmission mechanism, and a toilet. The test piece for urinalysis comprises a housing, a support, and test paper. The support is movably arranged on the housing, and the test paper is placed on the support to move with the support relative to the housing. In an initial state, the test paper is sealed in the housing. During testing, the test paper is exposed out of the housing by driving the support. The test paper can be effectively sealed to ensure its effectiveness and the accuracy of test results, and the test piece for urinalysis is more convenient and hygienic to use.

## Description

### TECHNICAL FIELD

The present invention relates to a test piece for urinalysis and a toilet with the test piece.

### BACKGROUND

In order to assist in the diagnosis of urinary system diseases and liver and gallbladder diseases, observe drug efficacy and safety, and evaluate health status, toilets with urinalysis functions are increasingly popular among consumers. At present, dozens of pieces of conventional urine routine test paper used for urinalysis toilets are mostly packaged in a bottle or box. Users often pay less attention to sealing the bottle or box after opening, or the bottle or box cannot be completely sealed after being opened. As a result, the unused test paper is damp, cannot be used for accurate testing, and can only be discarded and wasted. In addition, existing test paper needs to be soaked in urine and taken out from a device where the test paper is placed. However, the test paper soaked in urine is inconvenient to place and easily contaminates surrounding objects, making it inconvenient and unhygienic to use.

### SUMMARY

In order to solve the above problems, the present invention provides a test piece for urinalysis that can ensure effective sealing of test paper, effectiveness of the test paper and accuracy of test results and is more convenient and hygienic to use, and a toilet with the test piece.

One of the objectives of the present invention is to provide a test piece for urinalysis, installed on a toilet seat for human urinalysis, including a housing, a support, and test paper, where the support is movably arranged on the housing, and the test paper is placed on the support to move with the support relative to the housing; in an initial state, the test paper is sealed in the housing; during testing, the test paper is exposed out of the housing by driving the support.

Compared with the prior art, the beneficial effects of the above technical solution are as follows:
(1) The test paper for a single test is packaged in a sealed manner through the housing. On the one hand, the problem in the prior art that unused ones of the test paper placed together for multiple tests are prone to failure due to moisture is effectively avoided, and the accuracy of test results of the test paper can be ensured. On the other hand, the single piece of test paper is packaged in a sealed manner through the housing and placed on the support to move with the support relative to the housing, and the test paper does not need to be taken out from the support during testing, making the operation more convenient and hygienic.
(2) The support is movable between the extension position relative to the housing and the retraction position relative to the housing, so that the test paper can extend out of and retract into the housing with the support, and the test paper absorbing urine can retract into the housing by driving the support to avoid contaminating surrounding objects, further making the operation more convenient and hygienic.
(3) At least the portion of the side wall of the housing corresponding to the test paper is made of a transparent material, so that the test paper retracting into the housing can be directly observed and conveniently tested, with a simple structure.
(4) At least the portion of the side wall of the housing corresponding to the test paper is a straight wall, which can effectively avoid the influence of light on an optical instrument used for testing the test paper and ensure the accuracy of test results.
(5) The cross-sectional shape of the outer surface of the portion of the side wall of the housing that matches the test hole on the toilet seat matches the cross-sectional shape of the test hole, making the sealing between the side wall of the housing and the inner wall of the test hole easier and more reliable.

The second objective of the present invention is to provide another test piece for urinalysis, including a driving unit and a test unit, where the test unit includes a housing and a test element that extends and retracts relative to the housing, the housing is provided with a first opening, the driving unit can drive the test element to extend out of the housing from the first opening and move to an extension position, and the driving unit can also drive the test element to retract to a retraction position.

In this solution, the transmission fit between the driving unit and the test unit directly drives the test element to extend and retract, and the tested sample can be directly poured onto the test element during testing, so the test is more hygienic and convenient, with a simple structure and strong practicability.

The third objective of the present invention is to provide another test piece for urinalysis, including: a housing having a seal chamber and a first opening; a support, where test paper is provided on the support, the support is arranged in the seal chamber and extends and retracts relative to the housing through the first opening, a first sealing member is provided at a front end of the support, and the first sealing member is in sealing fit with the first opening when the support moves to a retraction position; the upper surface of the support is a mating surface for smooth transition, a push portion close to the mating surface is provided at the first opening, and the push portion can push a foreign matter on the mating surface during the retraction of the support.

In this solution, the upper surface of the support can achieve smooth transition. After the support is pushed out, even if the foreign matter falls onto the support, the foreign matter can be pushed away by the push portion when the support is pulled back, which ensures that the test results are not affected and the phenomenon of getting stuck does not occur. The test piece has a simple structure and stable functions.

The fourth objective of the present invention is to provide another test piece for urinalysis, including: a support having a limit portion; test paper provided with a plurality of test paper blocks used for contact reaction with to-be-tested liquid; and a gland having a pouring area and a plurality of isolation ribs, where the isolation ribs divide the pouring area into a plurality of isolation chambers, each of the isolation chambers corresponds to each of the test paper blocks, the isolation ribs isolate the adjacent test paper blocks, the gland is further provided with a clamping portion in clamping fit with the limit portion, and the gland is in clamping fit with the support to confine the test paper between the gland and the support.

In this solution, the gland is clamped with the support to confine the test paper between them, and the isolation ribs arranged on the gland isolate each test paper block to prevent color mixing between the test paper blocks, thereby improving the testing accuracy of the test paper.

The fifth objective of the present invention is to provide another test piece for urinalysis, including a test module, an identification module, and a driving unit, where the test module includes a support and test paper arranged on the support, and the test paper is provided with a plurality of test paper blocks; the identification module is configured to collect data from the test paper blocks, and the identification module includes at least one sensor and at least one light source; the identification module simultaneously tests at least two of the test paper blocks, the identification module further includes a light blocking plate, the light blocking plate is provided with emitting holes corresponding to the light sources and receiving holes corresponding to the sensors, and the driving unit is in transmission fit with the test module and/or the identification module.

In this solution, the identification module simultaneously tests at least two of the test paper blocks, and the driving unit moves the test module and/or the identification module, to identify and analyze each test paper block, thereby saving costs and achieving strong practicality and promotion.

The sixth objective of the present invention is to provide another test piece for urinalysis, arranged on a toilet, including: a base arranged on the toilet; a movable member capable of carrying a to-be-tested sample and movable along the base; and a test module arranged on the base, where the test module is movable relative to the movable member, and the test module includes a test state and a yield state; where in the test state, the test module is close to the movable member, the movable member carries the to-be-tested sample to a position corresponding to the test module, and the test module tests the to-be-tested sample; in the yield state, the test module moves to a position away from the movable member to yield the movable member.

In this solution, the test module is movable relative to the movable member, which can adjust the distance between the test module and the movable member according to different state requirements, thereby meeting different requirements for accurate test and movement of the movable member.

The seventh objective of the present invention is to provide a transmission mechanism for a test piece for urinalysis, including a driving unit and a test unit, where the test unit includes a housing and a test element that extends and retracts relative to the housing, the test element includes an isolation portion, a piece of test paper is installed on one side of the isolation portion, the other side of the isolation portion is mated with the driving unit, then the driving unit drives the test element to move to a preset position for testing, and the isolation portion isolates the test paper from the driving unit.

In this solution, the driving unit and the test unit are arranged independently, and the isolation portion isolates the driving unit from the test paper to prevent the driving unit from coming into contact with to-be-tested liquid, so the driving unit does not need to be cleaned, which does not affect the test results, with a simple structure.

The eighth objective of the present invention is to provide a segmented transmission mechanism for a test piece for urinalysis, including: a base having at least one installation portion; a test box having an accommodating chamber, where the test box is movably arranged at the installation portion; a test element used for testing to-be-tested liquid, where the test element is arranged in the accommodating chamber of the test box in an extensible and retractable manner; and a driving unit including a driving member, where the driving member is provided with a first driving portion and a second driving portion; the test element has a first mating portion in transmission fit with the first driving portion, and the test box has a second mating portion in transmission fit with the second driving portion; when the test element is in a retraction position, the second driving portion is separated from the second mating portion; after the driving member drives the test element to extend a preset distance through the first driving portion, the second driving portion abuts against the second mating portion for transmission fit.

The beneficial effects of this solution are as follows:
(1) One driving member drives the test element and the test box to move sequentially, with a simple structure and cost saving.
(2) The installation portion is provided with a first guide portion, the second driving portion can be in guide fit with the first guide portion, and the second driving portion moves along the first guide portion during the movement of the driving member, thereby avoiding affecting positioning accuracy by warping and bending during the movement of the driving member.
(3) The base is further provided with the limit portion, and the test box is provided with an abutting portion in limiting fit with the limit portion; when the test box moves to a preset position, the abutting portion abuts against the limit portion for limiting fit to limit the moving distance of the test box, thereby preventing the test box from falling out.

In addition, the present invention further provides a toilet, including the test piece for urinalysis as described in any of the above; or including the transmission mechanism for the test piece for urinalysis as described in any of the above; or including the segmented transmission mechanism for the test piece for urinalysis as described in any of the above.

### BRIEF DESCRIPTION OF THE DRAWINGS

The accompanying drawings described herein are intended to provide a further understanding of the present invention and form a part of the present invention. The illustrative embodiments of the present invention and the descriptions thereof are used for explaining the present invention and do not constitute undue limitation of the present invention. In the figures:
FIG. 1 is a longitudinal cross-sectional view of a test piece for urinalysis according to a first embodiment of the present invention, with a support in a retraction position;
FIG. 2 is a transverse cross-sectional view of the test piece for urinalysis according to the first embodiment of the present invention, with the support in the retraction position;
FIG. 3 is a longitudinal cross-sectional view of the test piece for urinalysis according to the first embodiment of the present invention, with the support in an extension position;
FIG. 4 is a transverse cross-sectional view of the test piece for urinalysis according to the first embodiment of the present invention, with the support in the extension position;
FIG. 5 is a schematic view of the test piece for urinalysis installed on a toilet seat according to the first embodiment of the present invention, with the support in the retraction position;
FIG. 6 is an enlarged view of part A in FIG. 5;
FIG. 7 is a schematic view of the test piece for urinalysis installed on the toilet seat according to the first embodiment of the present invention, with the support in the extension position;
FIG. 8 is an enlarged view of position B in FIG. 7;
FIG. 9 is a longitudinal cross-sectional view of a test piece for urinalysis according to a second embodiment of the present invention, with a support in a retraction position;
FIG. 10 is a longitudinal cross-sectional view of the test piece for urinalysis according to the second embodiment of the present invention, with the support in an extension position;
FIG. 11 is a perspective view of a test piece for urinalysis according to a third embodiment of the present invention, with a support in an extension position;
FIG. 12 is a longitudinal cross-sectional view of the test piece for urinalysis according to the third embodiment of the present invention, with the support in a retraction position;
FIG. 13 is a longitudinal cross-sectional view of the test piece for urinalysis according to the third embodiment of the present invention, with the support in the extension position;
FIG. 14 is a perspective view of a test piece for urinalysis according to a fourth embodiment of the present invention;
FIG. 15 is a cross-sectional view of the test piece for urinalysis according to the fourth embodiment of the present invention;
FIG. 16 is a first cross-sectional view of installation of the test piece for urinalysis and a toilet according to the fourth embodiment of the present invention;
FIG. 17 is a second cross-sectional view of installation of the test piece for urinalysis and the toilet according to the fourth embodiment of the present invention;
FIG. 18 is a perspective view of a test piece for urinalysis according to a fifth embodiment of the present invention;
FIG. 19 is a cross-sectional view of a test piece for urinalysis according to a sixth embodiment of the present invention;
FIG. 20 is a perspective view of a test piece for urinalysis according to a seventh embodiment of the present invention;
FIG. 21 is a first cross-sectional view of the test piece for urinalysis according to the seventh embodiment of the present invention;
FIG. 22 is a second cross-sectional view of the test piece for urinalysis according to the seventh embodiment of the present invention;
FIG. 23 is a first cross-sectional view of a toilet according to the seventh embodiment of the present invention;
FIG. 24 is a second cross-sectional view of the toilet according to the seventh embodiment of the present invention;
FIG. 25 is a cross-sectional view of another test piece for urinalysis according to the seventh embodiment of the present invention;
FIG. 26 is an assembly view of a test piece for urinalysis according to an eighth embodiment of the present invention;
FIG. 27 is an exploded view of the test piece for urinalysis according to the eighth embodiment of the present invention;
FIG. 28 is a first cross-sectional views of the test piece for urinalysis according to the eighth embodiment of the present invention;
FIG. 29 is a second cross-sectional view of the test piece for urinalysis according to the eighth embodiment of the present invention;
FIG. 30 is a first assembly view of a test piece for urinalysis according to a ninth embodiment of the present invention;
FIG. 31 is a second assembly view of the test piece for urinalysis according to the ninth embodiment of the present invention;
FIG. 32 is a cross-sectional view of the test piece for urinalysis according to the ninth embodiment of the present invention;
FIG. 33 is an assembly view of a test piece for urinalysis according to a tenth embodiment of the present invention;
FIG. 34 is a cross-sectional view of the test piece for urinalysis according to the tenth embodiment of the present invention;
FIG. 35 is an assembly view of a test piece for urinalysis according to an eleventh embodiment of the present invention;
FIG. 36 is a cross-sectional view of the test piece for urinalysis according to the eleventh embodiment of the present invention;
FIG. 37 is a schematic structural view of a test piece for urinalysis according to a twelfth embodiment of the present invention;
FIG. 38 is a schematic structural view of a test unit, a movable member, and a driving unit according to the twelfth embodiment of the present invention;
FIG. 39 is a schematic structural view of components in FIG. 37 when the test unit is in a to-be-tested state;
FIG. 40 is a schematic structural view of the components in FIG. 37 when the test unit is in a test state;
FIG. 41 is a schematic structural view of the components in FIG. 37 when the test unit is in a yield state;
FIG. 42 is a schematic structural view of a test unit, a movable member, and a driving unit according to a thirteenth embodiment of the present invention;
FIG. 43 is a cross-sectional view of assembly of a transmission mechanism for a test piece for urinalysis and a toilet according to a fourteenth embodiment of the present invention;
FIG. 44 is a schematic view of mating between a housing and a driving member of the transmission mechanism for the test piece for urinalysis according to the fourteenth embodiment of the present invention;
FIG. 45 is a schematic view of mating between a housing and a driving member of a transmission mechanism for a test piece for urinalysis according to a fifteenth embodiment of the present invention;
FIG. 46 is a cross-sectional view of a segmented transmission mechanism for a test piece for urinalysis according to a sixteenth embodiment of the present invention;
FIG. 47 is a cross-sectional view of a base of the segmented transmission mechanism for the test piece for urinalysis according to the sixteenth embodiment of the present invention;
FIG. 48 is a perspective view of a driving member of the segmented transmission mechanism for the test piece for urinalysis according to the sixteenth embodiment of the present invention;
FIG. 49 is a perspective view of a test box of the segmented transmission mechanism for the test piece for urinalysis according to the sixteenth embodiment of the present invention;
FIG. 50 is a first working state view of the segmented transmission mechanism for the test piece for urinalysis according to the sixteenth embodiment of the present invention;
FIG. 51 is a second working state view of the segmented transmission mechanism for the test piece for urinalysis according to the sixteenth embodiment of the present invention;
FIG. 52 is a third working state view of the segmented transmission mechanism for the test piece for urinalysis according to the sixteenth embodiment of the present invention; and
FIG. 53 is a perspective view of a driving member of a segmented transmission mechanism for a test piece for urinalysis according to a seventeenth embodiment of the present invention.

### Explanation of reference numerals:

1. Housing; 11. First opening; 111. Push portion; 12. Second opening; 13. Straight wall; 14. Portion of the side wall of the housing that matches the test hole; 15. Seal chamber; 16. Blocking surface;
2. Support; 21. Driving portion; 22. Gland; 221. Mating surface; 222. Isolation rib; 223. Isolation chamber; 224. Buckle; 225. Drainage notch; 226. Fool-proof clamping groove; 227. First mating portion; 228. Baffle; 23. Convex rib; 24. Fool-proof rib; 25. Limit member; 26. Test probe; 251. Guide groove; 27. Guide rail; 28. Isolation portion;
3. Test paper; 31. Test paper block; 4. First sealing member; 5. Second sealing member; 6. First sealing film; 7. Second sealing film; 8. Ejector pin; 9. First magnetic member; 10. Second magnetic member; 70. Casing; 80. Transmission belt; 90a. First magnetic member; 90b. Second magnetic member;
100. Toilet seat; 101. Test hole; 102. Installation hole; 103. Nozzle assembly; 104. Base; 1041. Through hole; 1042. Accommodating chamber; 1043. Sleeve; 1044. Installation chamber; 1045. First chute; 1046. Fixed joint; 1047. Hollow channel; 1048. Second chute; 1049. Limit portion;
200. Electric drive mechanism; 201. Motor; 202. Gear; 203. Rack; 204. First driving portion; 205. Second driving portion;
300. Identification module; 321. Sensor; 322. Light source; 323. Light blocking plate; 3231. Emitting hole; 3232. Receiving hole; 324. Circuit board;
400. Movable member; 431. Limit rib;
500. Test box; 521. Accommodating chamber; 522. Second mating portion; 523. Second guide portion; 524. Abutting portion; 525. Boss.

### DETAILED DESCRIPTION

In order to make the technical problems to solved, technical solutions and advantages of the present invention clearer, the present invention will be further described in detail below in conjunction with the accompanying drawings and embodiments. It should be understood that the specific embodiments described herein are merely used for interpreting the present invention, rather than limiting the present invention.

### First embodiment:

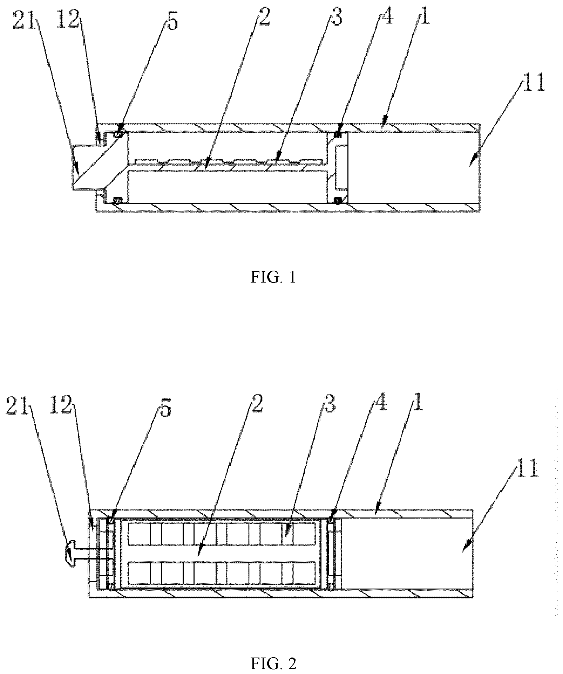

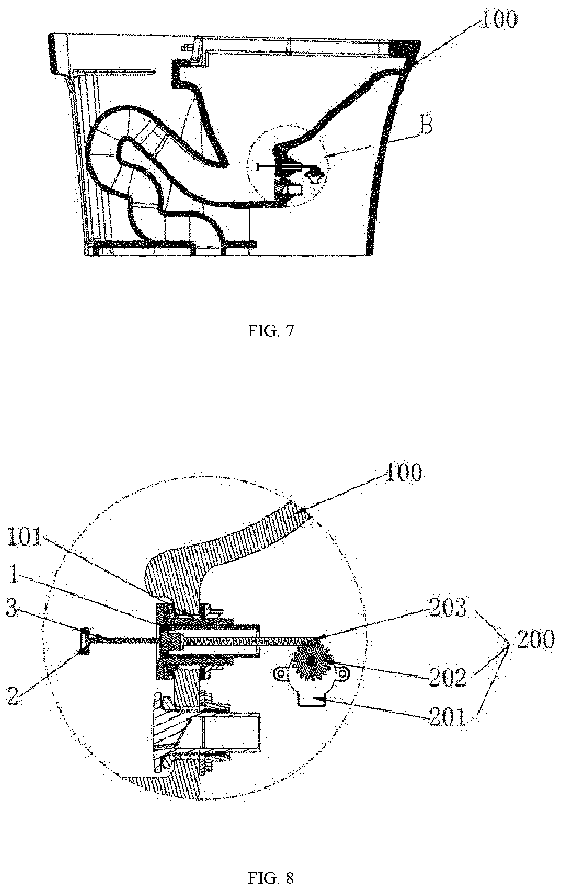

As shown in FIG. 1 to FIG. 8, a test piece for urinalysis in this embodiment is installed on a toilet seat 100 of a toilet for human urinalysis. The test piece for urinalysis includes a housing 1, a support 2, and test paper 3. The support 2 is movably arranged on the housing 1, and the test paper 3 is placed on the support 2 to move with the support 2 relative to the housing 1. In an initial state, the test paper 3 is sealed in the housing 1. During testing, the test paper 3 is exposed out of the housing 1 by driving the support 2 and can come into contact with urine. The test paper 3 for a single test is packaged in a sealed manner through the housing 1. On the one hand, the problem in the prior art that unused ones of the test paper placed together for multiple tests are prone to failure due to moisture is effectively avoided, and the accuracy of test results of the test paper can be ensured. On the other hand, the single piece of test paper 3 is packaged in a sealed manner through the housing 1, the test paper 3 is placed on the support 2 to move with the support 2 relative to the housing 1, and the test paper 3 does not need to be taken out from the support 2 during testing, making the operation more convenient and hygienic.

In this embodiment, the support 2 is arranged on the housing 1 in an extensible and retractable manner. The housing 1 is provided with a first opening 11 for the support 2 to extend and retract relative to the housing 1 and a second opening 12 for driving the support 2. The support 2 is movable between an extension position relative to the housing 1 and a retraction position relative to the housing 1.

In the initial state, the support 2 is in the retraction position, and a seal chamber for sealing the test paper 3 is formed between the support 2 and the housing 1, or both the support 2 and the test paper 3 are arranged in a seal chamber formed by an inner cavity of the housing 1. This embodiment adopts the former sealing method. Specifically, as shown in FIG. 1 and FIG. 2, the sealing method for the seal chamber in this embodiment is as follows: a first sealing member 4 and a second sealing member 5 are provided between the support 2 and the housing 1; when the support 2 is in the retraction position, the second opening 12 is sealed by the second sealing member 5 between the support 2 and the housing 1, the first sealing member 4 is in sealing fit with an inner wall of the housing 1, and the end of the support 2 provided with the first sealing member 4 seals and separates the inner cavity of the housing 1 into two chambers, where the chamber away from the first opening 11 forms the seal chamber for accommodating the test paper 3. Alternatively, in other embodiments, the sealing position of the first sealing member 4 may be arranged at the first opening 11 or nearby the first opening 11, the first opening 11 is sealed by the first sealing member 4 between the support 2 and the housing 1, and the seal chamber for accommodating the test paper 3 is formed by sealing the first opening 11 and the second opening 12.

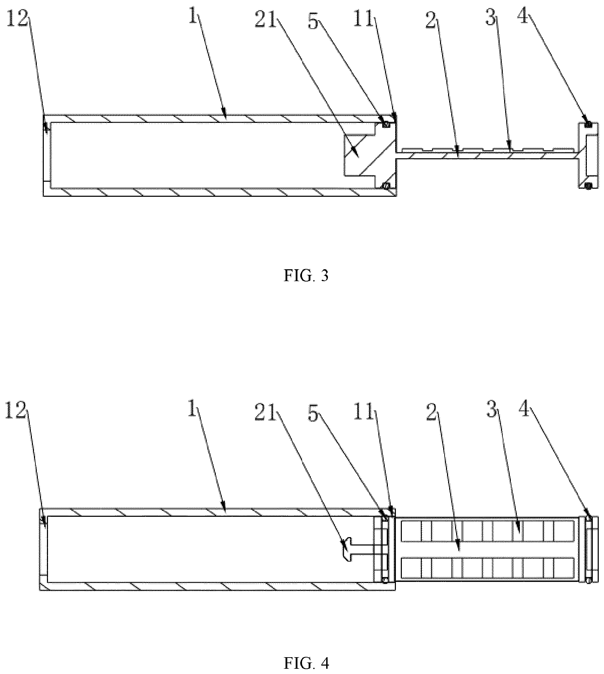

As shown in FIG. 3 and FIG. 4, during testing, the support 2 is driven, so that the support 2 is located in the extension position, and the test paper 3 is exposed out of the seal chamber from the first opening 11 to absorb urine.

Preferably, in this embodiment, the first opening 11 and the second opening 12 are arranged opposite, and a driving portion 21 is provided at the end of the support 2 close to the second opening 12. When the support 2 is in the retraction position, the driving portion 21 is located outside the housing 1, which facilitates manual driving of the driving portion 21, or facilitates the connection or disconnection of the driving portion 21 and an external drive mechanism.

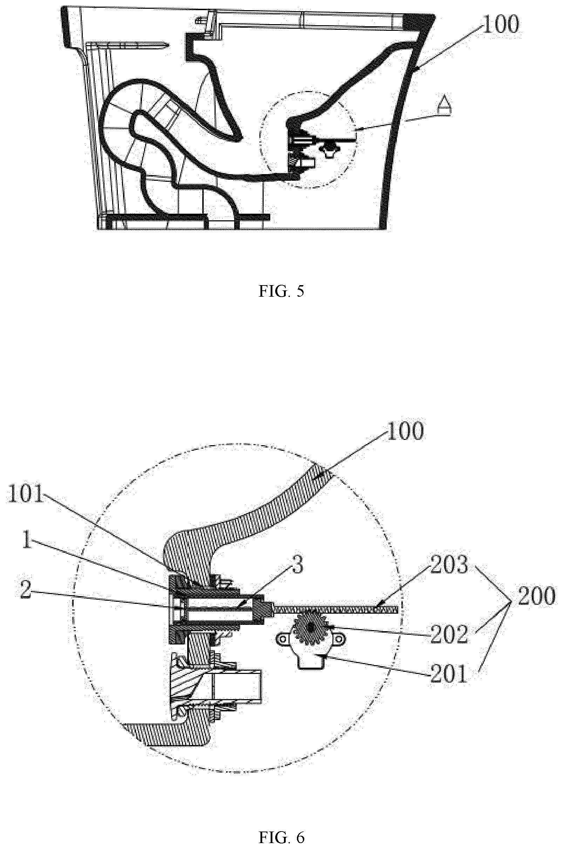

The support 2 may be directly driven by hand or driven by an electric drive mechanism, which is not limited here. As shown in FIG. 5 to FIG. 8, the support 2 is driven by an electric drive mechanism 200 in this embodiment. The electric drive mechanism 200 may include a motor 201, a gear 202, and a rack 203. The motor 201 drives the gear 202, the gear 202 drives the rack 203, the rack 203 drives the support 2, and the rack 203 is provided with a connection portion (not shown) detachably connected to the driving portion 21. Alternatively, in other embodiments, when the electric drive mechanism is used for driving, the electric drive mechanism may include a motor, a synchronous pulley, and a synchronous belt, where the motor drives the synchronous pulley, the synchronous pulley drives the synchronous belt, and the synchronous belt drives the support.

In this embodiment, the electric drive mechanism 200 directly drives the support 2 to retract into the housing 1, with a simple structure. Alternatively, in other embodiments, the support 2 may not be connected to the rack 203, the rack 203 is merely used for driving the support 2 to extend out of the housing 1, and the support 2 is reset to the retraction position by an elastic force of an elastic member (not shown). Specifically, the elastic member is provided between the housing 1 and the support 2. The support 2 extends to the extension position by overcoming the elastic force of the elastic member under an external driving force (namely, the driving force of the electric drive mechanism 200). When the external driving force applied to the support 2 is removed, the support 2 is reset to the retraction position under the elastic force of the elastic member. The elastic member may specifically be a spring.

In this embodiment, at least the portion of the side wall of the housing 1 corresponding to the test paper 3 is made of a transparent material. The housing 1 may be made entirely of the transparent material, or only the portion corresponding to the test paper 3 may be made of the transparent material. In this case, the test paper 3 retracting into the housing 1 can be directly observed and conveniently tested, with a simple structure. The transparent material for manufacturing the housing 1 may be acrylic or the like.

As shown in FIG. 5 to FIG. 8, in this embodiment, the toilet seat 100 is provided with a test hole 101, and the test hole 101 is used for mating with the test piece for urinalysis as described in any of the above. The test piece is detachably arranged at the test hole 101, so that the entire test piece can be directly discarded after each test, a new test piece can be used for the next test, and the test hole 101 can be blocked with a plug (not shown) in a non-test state.

The cross-sectional shape of the outer surface of the portion of the side wall of the housing 1 that matches the test hole 101 on the toilet seat 100 matches the cross-sectional shape of the test hole, making the sealing between the side wall of the housing 1 and the inner wall of the test hole 101 easier and more reliable. In this embodiment, the housing 1 is of a cylindrical structure with two open ends, and the test hole 101 is a circular hole.

### Second embodiment:

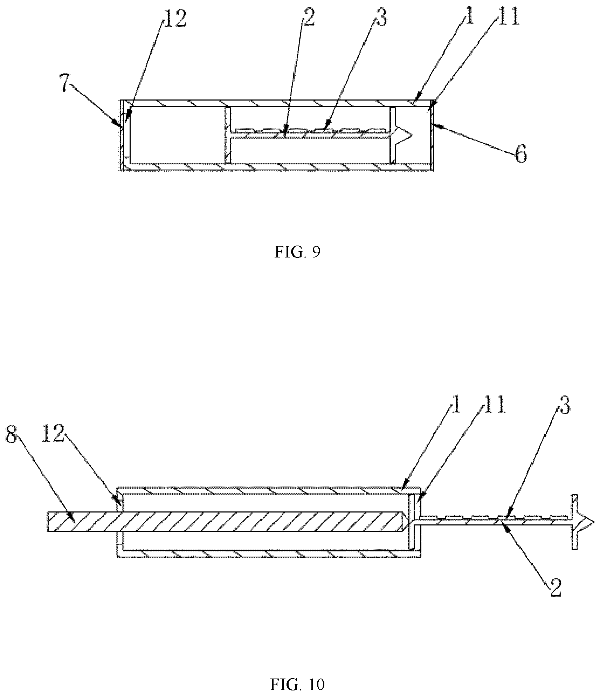

As shown in FIG. 9 and FIG. 10, a test piece for urinalysis in this embodiment differs from the first embodiment mainly in the sealing method for the test paper 3 and the driving method for the support 2.

Specifically, in this embodiment, the first opening 11 is sealed by a penetrable first sealing film 6, the second opening 12 is sealed by a penetrable second sealing film 7, the inner cavity of the housing 1 forms a seal chamber through the first sealing film 6 and the second sealing film 7, and the first sealing film 6 and the second sealing film 7 seal the support 2 and the test paper 3 in the seal chamber formed by the inner cavity of the housing 1. The first sealing film 6 and the second sealing film 7 may be aluminum foils, rubber parts, plastic sheets, or the like.

In this embodiment, an ejector pin 8 penetrates the second sealing film 7 to drive the support 2, and the support 2 then penetrates the first sealing film 6 to extend out of the housing 1. The ejector pin 8 can be directly driven by hand or driven by an electric drive mechanism.

### Third embodiment:

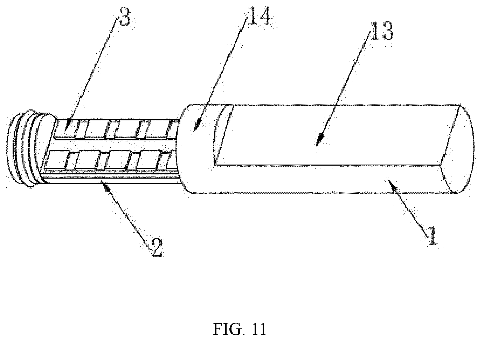

As shown in FIG. 11 to FIG. 13, a test piece for urinalysis in this embodiment differs from the first embodiment mainly in the sealing method for the test paper 3 and the driving method for the support 2.

Specifically, in this embodiment, the housing 1 is provided with only the first opening 11, but not provided with the second opening 12 for driving the support 2. In this embodiment, the support 2 is driven in a non-contact manner. Specifically, a first magnetic member 9 is installed on the support 2, and the support 2 is driven by a magnetic force between an external magnetic drive component and the first magnetic member 9 on the support 2. The magnetic drive component is specifically a second magnetic member 10. The support 2 is driven by the magnetic force formed between the first magnetic member 9 and the second magnetic member 10, and there is an attractive magnetic force or repellent magnetic force between the first magnetic member 9 and the second magnetic member 10. The second magnetic member 10 may alternatively be replaced with an electromagnet (not shown), and there is an attractive magnetic force or repellent magnetic force between the energized electromagnet and the first magnetic member 9.

In this embodiment, the first opening 11 may be sealed by the first sealing member 4 in the first embodiment or the first sealing film 6 in the second embodiment.

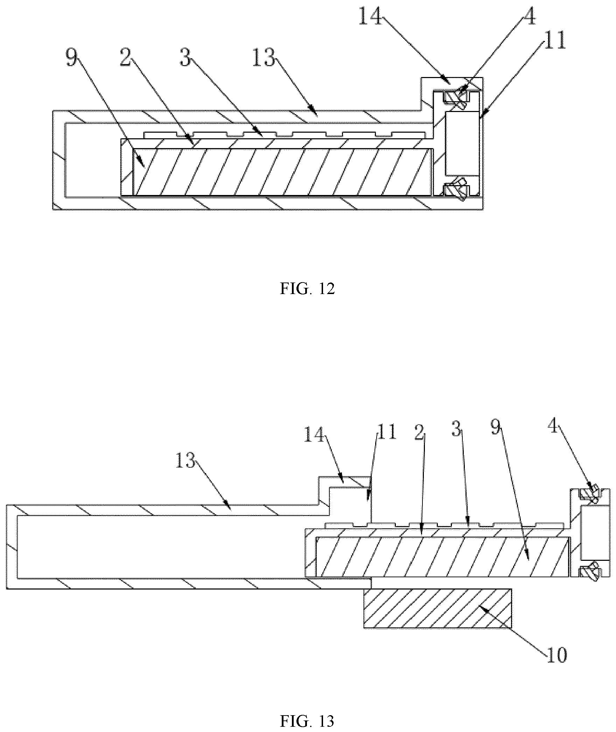

FIG. 12 shows a state of the support 2 in the retraction position, and FIG. 13 shows a state of the support 2 in the extension position under the drive of the second magnetic member 10.

Moreover, in this embodiment, the housing 1 is not of a cylindrical structure, but at least the portion of the side wall of the housing 1 corresponding to the test paper 3 is designed as a straight wall 13. When the test paper is tested with an optical instrument, the straight wall 13 can effectively avoid the influence of light on the optical instrument and ensure the accuracy of test results. In order to make the sealing between the housing 1 and the test hole 101 easier and more reliable, the cross-sectional shape of the outer surface of the portion 14 of the side wall of the housing 1 that matches the test hole 101 on the toilet seat 100 matches the cross-sectional shape of the test hole, both of which are circular cross-sections.

Notably, in the first embodiment and the second embodiment, the housing 1 may not be provided with the second opening 12, and the support 2 can also be driven in a non-contact manner such as by magnetic force.

### Fourth embodiment:

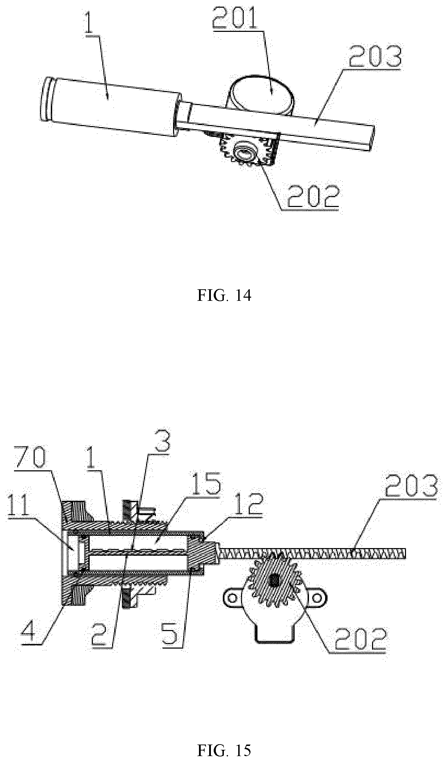

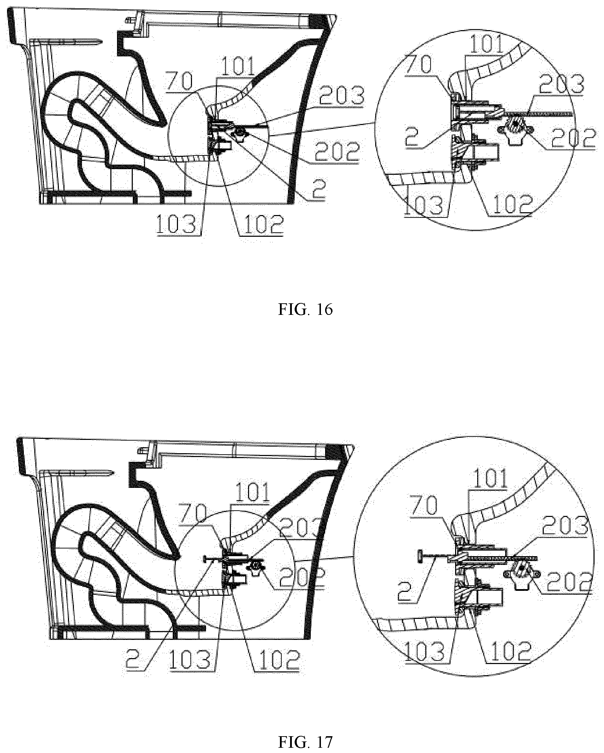

As shown in FIG. 14 to FIG. 17, a test piece for urinalysis includes a driving unit and a test unit, the test unit includes a housing 1 and a test element that extends and retracts relative to the housing 1, the housing 1 is provided with a first opening 11, the driving unit is in transmission fit with the test element, the driving unit can drive the test element to extend out of the housing 1 from the first opening 11 and move to an extension position, and the driving unit can also drive the test element to retract to a retraction position.

In this embodiment, the housing 1 has a seal chamber 15, the test element is arranged in the seal chamber 15, the test unit includes a first sealing member 4, the first sealing member 4 can be in sealing fit with the first opening 11 to seal the test element in the seal chamber 15, the first sealing member 4 is a rubber part or a sealing film, and the sealing film may be an aluminum foil.

In this embodiment, the first sealing member 4 is a sealing ring arranged at one end of the test element. When the test element is in the retraction position, the first sealing member 4 is in sealing fit with the inner wall of the seal chamber 15 close to the first opening 11.

In other embodiments, the first sealing member 4 may be in sealing fit with the first opening 11.

In this embodiment, the housing 1 is further provided with a second opening 12, and the test element extends from the second opening 12 to connect with the driving unit, or the driving unit extends from the second opening 12 into the housing 1 to connect with the test element. The former is specifically used in this embodiment.

In this embodiment, the test unit further includes a second sealing member 5, the test element is located between the first sealing member 4 and the second sealing member 5, the second sealing member 5 can be in sealing fit with the inner wall of the seal chamber 15 close to the second opening 12, and the second sealing member 5 is a rubber part or a sealing film.

In this embodiment, the second sealing member 5 is a sealing ring arranged at the other end of the test element. When the test element is in the retraction position, the second sealing member 5 is in sealing fit with the inner wall of the seal chamber 15 close to the second opening 12.

In other embodiments, the second sealing member 5 may be in sealing fit with the second opening 12.

In this embodiment, the test element includes a support 2 and test paper 3, the test paper 3 is placed on the support 2, and the driving unit is in transmission fit with the support 2. Specifically, the first sealing member 4 and the second sealing member 5 are located at two ends of the support 2.

In this embodiment, the housing 1 is a hollow sleeve.

In this embodiment, the driving unit includes a driving member in transmission fit with the test unit, and a power source that provides driving force to the driving member.

In this embodiment, the power source is a water pressure source, a pneumatic pressure source, a magnetic component, or a motor 201.

In this embodiment, the driving member includes a gear 202 and a rack 203, or the driving member includes a transmission belt 80.

In this embodiment, the driving member includes a gear 202 and a rack 203 that is in transmission fit with the support 2, the power source is a motor 201, and the motor 201 drives the gear 202 to rotate.

This embodiment further provides a toilet, including the test piece for urinalysis as described in any one of the above, where the bottom of a toilet bowl is provided with a test hole 101, and the housing 1 is detachably or flexibly installed at an installation hole 60a. Specifically, a casing 70 is fixedly installed at the test hole 101, and the housing 1 is detachably or flexibly installed inside the casing 70.

In this embodiment, an installation hole 102 is further provided at the bottom of the toilet bowl, a nozzle assembly 103 is installed inside the installation hole 102, and the nozzle assembly 103 sprays water to the bottom of the toilet bowl.

When the toilet is in normal use, the test unit is located below a water seal surface of the toilet. When a test is prepared, the nozzle assembly 103 sprays water to the bottom of the toilet bowl, a sewage pipe of the toilet forms a siphon, the water in the water seal is extracted through the siphon effect, the water level in the water seal is lower than the test unit, and then the driving unit drives the support 2 to move to the extension position to prevent contaminating the test element. After the detected sample flows down along the inner wall of the toilet bowl, comes into contact with the test paper 3 and reacts, the support 2 is driven to retract into the housing 1, so that the first sealing member 4 is in sealing fit with the first opening 11, and the second sealing member 5 is in sealing fit with the second opening 12.

In this solution, the transmission fit between the driving unit and the test unit directly drives the test element to extend and retract, and the tested sample can be directly poured onto the test element during testing, so the test is more hygienic and convenient, with a simple structure and strong practicability.

### Fifth embodiment:

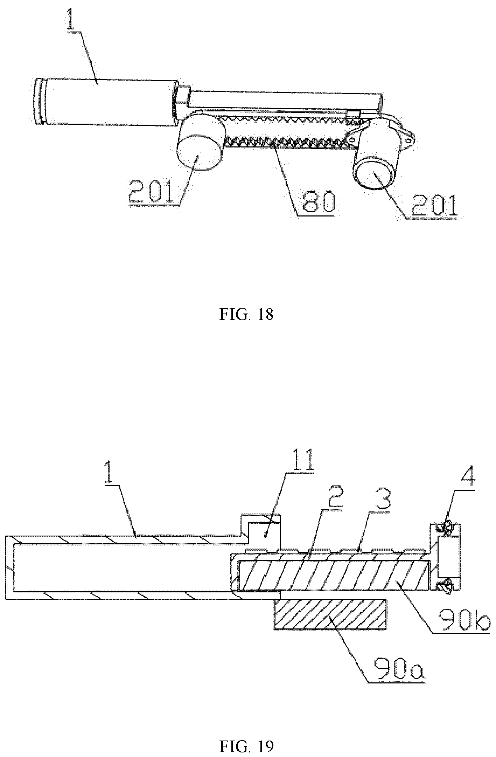

As shown in FIG. 18, this embodiment mainly differs from the fourth embodiment in that the driving member in this embodiment includes a transmission belt 80, the transmission belt 80 is provided with transmission teeth, and the power source is a motor 201 with a gear. The other unspecified parts are consistent with the fourth embodiment and will not be repeated here.

### Sixth embodiment:

As shown in FIG. 19, this embodiment mainly differs from the fourth embodiment in that the power source in this embodiment includes a magnetic assembly, which includes a first magnetic member 90a movably arranged outside the housing and a second magnetic member 90b fixedly arranged on the test element. Specifically, the second magnetic member 90b is arranged at the bottom of the support 2, and a magnetic force is produced between the first magnetic member 90a and the second magnetic member 90b to drive the test element to extend and retract.

The other unspecified parts are consistent with the fourth embodiment and will not be repeated here.

### Seventh embodiment:

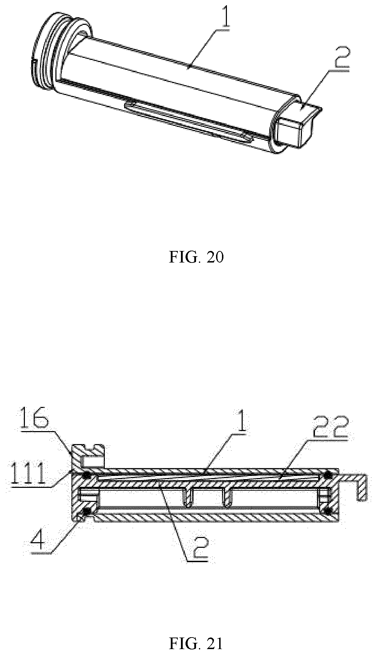

As shown in FIG. 20 to FIG. 22, a test piece for urinalysis includes:
a housing 1 having a seal chamber 15 and a first opening 11; and
a support 2, where test paper is provided on the support 2, the support 2 is arranged in the seal chamber 15 and extends and retracts relative to the housing 1 through the first opening 11, a first sealing member 4 is provided at a front end of the support 2, and the first sealing member 4 is in sealing fit with the first opening 11 when the support 2 moves to a retraction position;
where the upper surface of the support 2 is a mating surface 221 for smooth transition, a push portion 111 close to the mating surface 221 is provided at the first opening 11, and the push portion 111 can push a foreign matter on the mating surface 221 during the retraction of the support 2.

In this embodiment, the upper wall of the seal chamber 15 is parallel to the mating surface 221, and the upper wall of the seal chamber 15 is close to the mating surface 221.

In this embodiment, the mating surface 221 is a straight or curved surface, and the seal chamber 15 is cylindrical with a straight or curved upper wall.

In this embodiment, the support 2 is provided with a sink, a convex portion higher than the sink is provided at the front end of the support 2, and the first sealing member 4 is arranged on the convex portion. Specifically, the first sealing member 4 is a sealing ring sleeved on the convex portion.

In this embodiment, the test paper is laid on the sink, the test piece for urinalysis further includes a gland 22, the gland 22 presses the test paper onto the support 2, and an upper surface of the gland 22 and an upper surface of the convex portion B have the same height and are mated to form the mating surface 221.

In this embodiment, the upper wall of the first opening 11 forms the push portion.

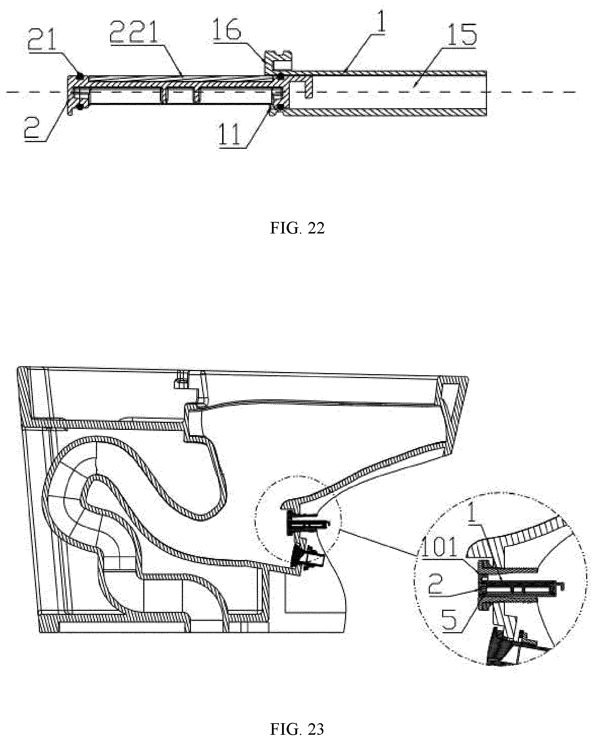

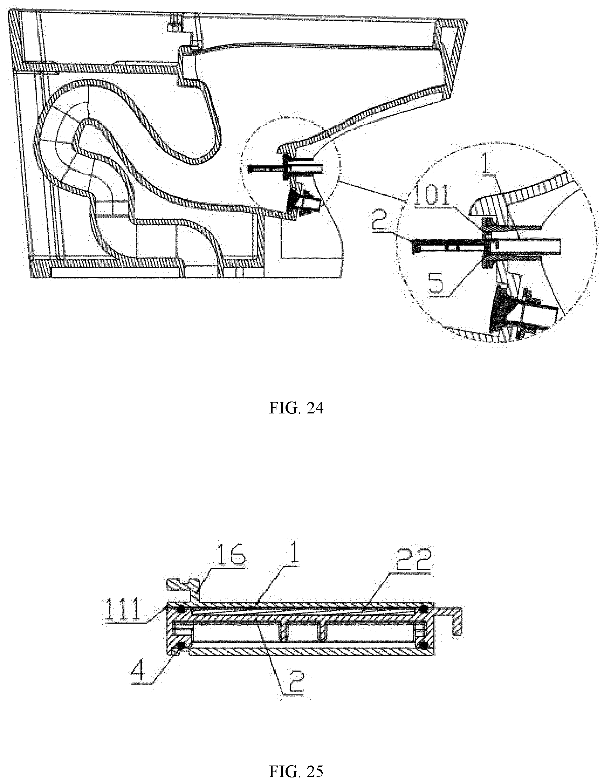

As shown in FIG. 23 and FIG. 24, this embodiment further provides a toilet including the aforementioned test piece for urinalysis, where the bottom of the toilet is provided with a test hole 101, and the housing 1 is arranged at the test hole 101. Specifically, the housing 1 can extend and retract relative to the test hole 101.

In this embodiment, there is a spacing gap between the upper surface of the housing 1 and the test hole 101, a blocking surface 16 extending upwards is provided at the front end of the housing 1, and the blocking surface 16 is used for filling the spacing gap.

In this embodiment, the blocking surface 16 and the push portion 111 are located on the same plane.

In other embodiments, as shown in FIG. 25, the blocking surface 16 is misaligned with the push portion 111. Specifically, the blocking surface 16 is located behind the push portion 111.

In this embodiment, a third sealing member in sealing fit with the test hole 101 is provided on the housing 1 close to the blocking surface 16. Specifically, the third sealing member is a sealing ring.

In this solution, the upper surface of the support 2 can achieve smooth transition. After the support 2 is pushed out, even if the foreign matter falls onto the support 2, the foreign matter can be pushed away by the push portion 111 when the support 2 is pulled back, which ensures that the test results are not affected and the phenomenon of getting stuck does not occur. The test piece has a simple structure and stable functions.

### Eighth embodiment:

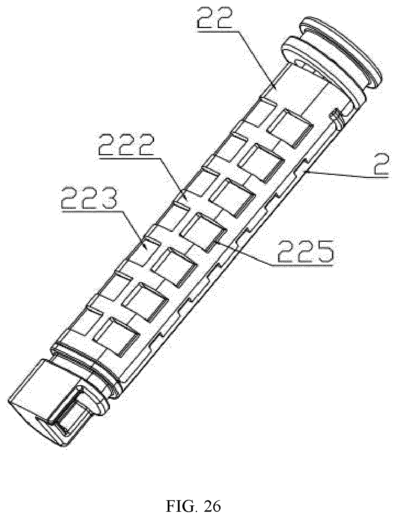

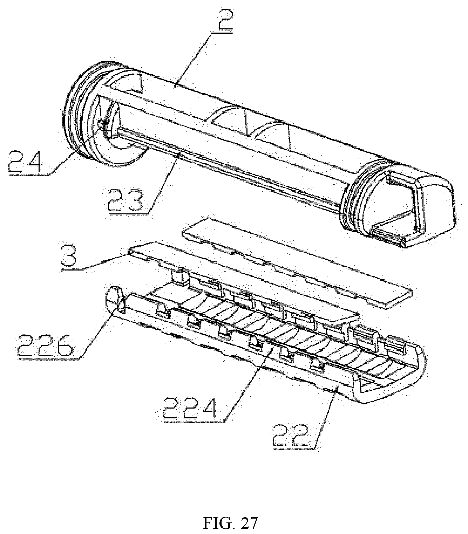

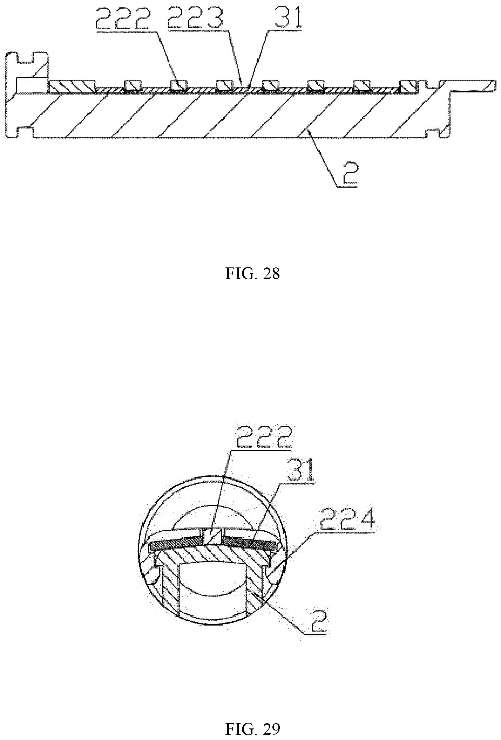

As shown in FIG. 26 to FIG. 29, a test piece for urinalysis includes:
a support 2 having a limit portion;
test paper 3, where the test paper 3 is provided with a plurality of test paper blocks 31, and the test paper blocks 31 are used for contact reaction with to-be-tested liquid; and
a gland 22 having a pouring area and a plurality of isolation ribs 222, where the isolation ribs 222 divide the pouring area into a plurality of isolation chambers 223, each of the isolation chambers 223 corresponds to each of the test paper blocks 31, the isolation ribs 222 isolate the adjacent test paper blocks 31, the gland 22 is further provided with a clamping portion in clamping fit with the limit portion, and the gland 22 is in clamping fit with the support 2 to confine the test paper 3 between the gland 22 and the support 2.

In this embodiment, the clamping portion includes a plurality of buckles 224 spaced apart in a length direction of the gland 22, and the limit portion includes convex ribs 23 extending in a length direction of the support 2.

In this embodiment, the buckles 224 are arranged at the positions corresponding to the isolation chambers 223. Further, each of the buckles 224 corresponds to each of the isolation chambers 223, and the length of the buckles 224 is the same as the size of the isolation chambers 223.

In this embodiment, the side of the isolation chamber 223 adjacent to the outside of the pouring area has a drainage notch 225, and then the to-be-tested liquid can overflow from the drainage notch 225 to the outside.

In this embodiment, the side of the isolation chamber 223 adjacent to the outside of the pouring area is an outer wall, an inner wall of the isolation chamber 223 and a front wall and a rear wall of the isolation chamber 223 are all higher than the outer wall, and then an upper area of the outer wall forms the drainage notch 225.

In this embodiment, the inner wall of the isolation chamber 223 and the front wall and rear wall of the isolation chamber 223 are all higher than the test paper block 31 to prevent liquid leakage between the isolation chambers 223, and the shape and size of the isolation chambers 223 match the test paper blocks 31.

In this embodiment, the isolation ribs 222 include a plurality of transverse ribs arranged in a width direction of the gland 22 and longitudinal ribs arranged in the length direction of the gland 22, and the transverse ribs intersect and match the longitudinal ribs to separate the plurality of isolation chambers 223. Specifically, the isolation chambers 223 are arranged in two columns in the length direction of the gland 22.

In this embodiment, the isolation chamber 223 penetrates an upper surface and a lower surface of the gland 22, and the drainage notch 225 is arranged on an outer side of the isolation chamber 223. Specifically, the cross-sections of both the support 2 and the gland 22 are gradually inclined downwards from the middle to two sides, and the test paper 3 is installed on inclined surfaces on two sides in a downward inclined state. When the test paper blocks 31 cannot absorb the to-be-tested liquid in time, excess to-be-tested liquid can quickly flow away from the isolation chambers 223 to prevent liquid leakage between the isolation chambers 223.

In this embodiment, one of the support 2 and the gland 22 is provided with fool-proof ribs 24, the other of the support 2 and the gland 22 is provided with fool-proof clamping grooves 226, and the fool-proof ribs 24 are mated with the fool-proof clamping grooves 226. Specifically, the shape and size of the fool-proof ribs 24 and the fool-proof clamping grooves 226 are the same, thereby preventing the gland 22 from being installed upside down.

In this embodiment, the test paper blocks 31 are color blocks corresponding to different human health indicators.

When installed, the test paper 3 is first placed inside the gland 22, each test paper block 31 is installed in one-to-one correspondence with each isolation chamber 223, and then the support 2 is fastened with the gland 22 to fix the test paper 3 between the gland 22 and the support 2. When in use, urine uniformly contacts and reacts with each test paper block 31 through the isolation chambers 223.

In this solution, the gland 22 is fastened with the support 2 to fix the test paper 3 between the gland 22 and the support 2, and the isolation ribs 222 arranged on the gland 22 isolate each test paper block 31 to prevent color mixing between the test paper blocks 31, thereby improving the testing accuracy of the test paper.

### Ninth embodiment:

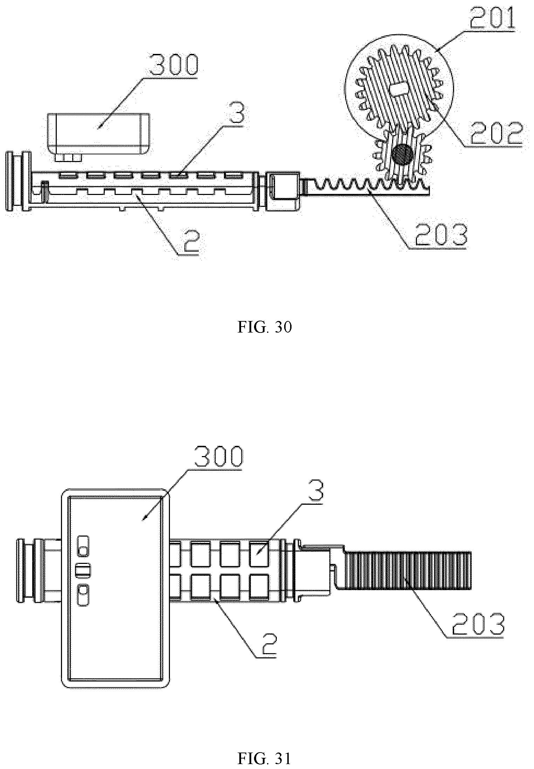

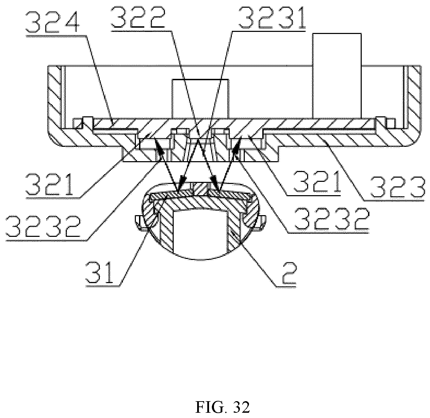

As shown in FIG. 30 to FIG. 32, a test piece for urinalysis includes a test module, an identification module 300, and a driving unit, where the test module includes a support 2 and test paper 3 arranged on the support 2, and the test paper 3 is provided with a plurality of test paper blocks 31; the identification module 300 is configured to collect data from the test paper blocks 31, and the identification module 300 includes at least one sensor 321 and at least one light source 322; the identification module simultaneously tests at least two of the test paper blocks, the identification module 300 further includes a light blocking plate 323, the light blocking plate 323 is provided with an emitting hole 3231 corresponding to the light source 322 and a receiving hole 3232 corresponding to the sensor 321, and the driving unit is in transmission fit with the test module and/or the identification module 300.

In this embodiment, the test paper blocks 31 are color blocks that reflect different health indicators.

In this embodiment, the identification module 300 includes two sensors 321 and one light source 322, and the light source 322 is located between the two sensors 321. When working, light from the light source 322 is irradiated to two test paper blocks 31 and then reflected by the two test paper blocks 31 to the two sensors 321 separately.

In other embodiments, the identification module 300 includes one sensor 321 and two light sources 322, the sensor 321 is located between the two light sources 322, and light from the two light sources 322 is irradiated to two test paper blocks 31 and then reflected by the two test paper blocks 31 to the sensor 321 separately.

In this embodiment, the test paper blocks 31 are arranged in two rows in the length direction of the support 2, and the two sensors 321 correspond one to one with the two test paper blocks 31 in the width direction of the support 2.

In this embodiment, the driving unit and the test module and/or the identification module 300 move in the length direction of the support 2, and the spacing between the test paper blocks 31 arranged in the length direction of the support 2 is equal.

In this embodiment, the quantity of emitting holes 3231 is the same as that of light sources 322, and the emitting holes 3231 correspond one to one with the light sources 322. The quantity of receiving holes 3232 is the same as that of sensors 321, and the receiving holes 3232 correspond one to one with the sensors 321. Specifically, the emitting holes 3231 gradually widen in a direction of light emission, and the receiving holes are light guide holes used for guiding light to the sensors.

In this embodiment, both the emitting holes 3231 and the receiving holes 3232 are sealed by transparent members.

In this embodiment, the sensor 321 is a color sensor, the light source 322 is an LED light, the test piece for urinalysis further includes a circuit board 324, and the color sensor and the LED light are both arranged on the circuit board 324. Specifically, the LED light emits high-brightness white light.

In this embodiment, the driving unit includes a rack 203 in transmission fit with the support 2 and further includes a motor 201 and a gear set 202, where the motor 201 drives the rack 203 to move through the gear set 202.

When working, the LED light (light source 322) is turned on, the light is irradiated to the test paper blocks 31 on two sides and then reflected by the test paper blocks 31 to the sensors 321 on two sides, the sensors 321 obtain information of the light and deduce health data of the corresponding test paper blocks 31, then the driving unit drives the support 2 to move and switch to correspond with the identification device for the next group of test paper blocks 31 to identify the next group of test paper blocks 31, and so on, until all the test paper blocks 31 are identified.

In this solution, at least two sensors 321 correspond one to one with at least two adjacent test paper blocks 31, and the driving unit moves the test module and/or the identification module 300, to identify and analyze each test paper block 31, thereby saving costs and achieving strong practicality and promotion.

### Tenth embodiment:

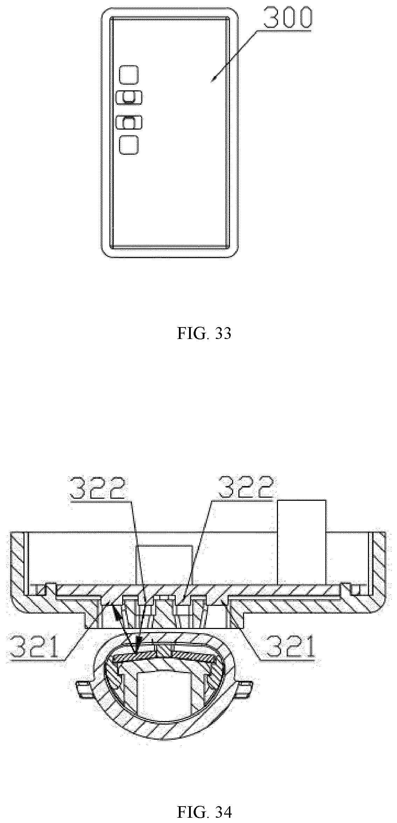

As shown in FIG. 33 and FIG. 34, this embodiment mainly differs from the ninth embodiment in that, in this embodiment, the quantity of light sources 322 is the same as that of sensors 321, and each light source 322 is arranged in one-to-one correspondence with each sensor 321.

In this embodiment, the light sources 322 and the sensors 321 are arranged in the left and right direction of the test paper blocks 31. The other unspecified parts are consistent with the ninth embodiment and will not be repeated here.

### Eleventh embodiment:

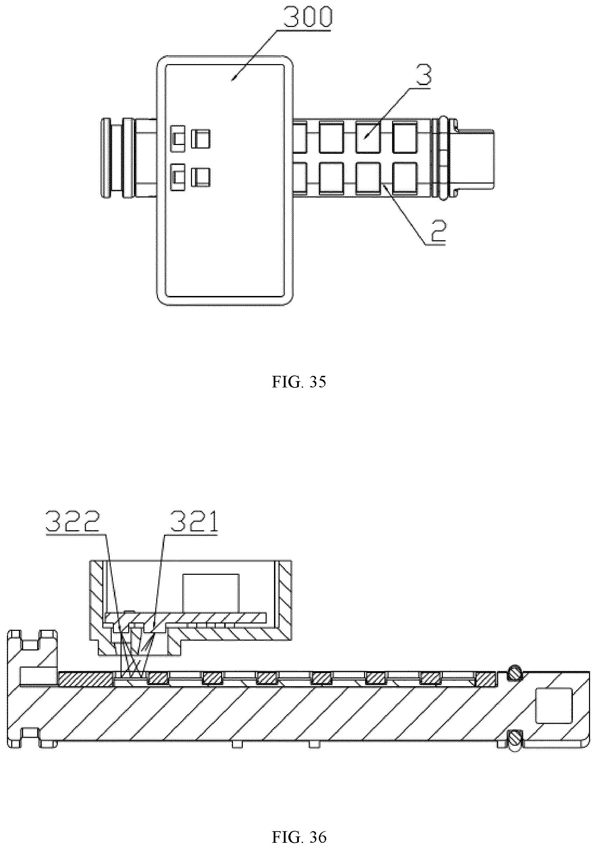

As shown in FIG. 35 and FIG. 36, this embodiment mainly differs from the ninth embodiment in that the light sources 322 and the sensors 321 are arranged in the front and rear direction of the test paper blocks. The other unspecified parts are consistent with the tenth embodiment and will not be repeated here.

### Twelfth embodiment:

In related technologies, a test piece for urinalysis is installed on a toilet, and the test piece for urinalysis needs to extract a to-be-tested sample from the toilet for testing. Therefore, a channel is provided between the test piece for urinalysis and the toilet to allow an extraction member to extend into the toilet for extraction. In view of a state of extracting the to-be-tested sample, a to-be-tested state and a test state, as well as different distance requirements between a test module and a movable member that extracts the to-be-tested sample, the key problem to be solved in this embodiment is how to adjust the distance to meet the requirements for test accuracy, activity, sealing, etc. in different states.

This embodiment provides a test piece for urinalysis, which is arranged on a toilet.

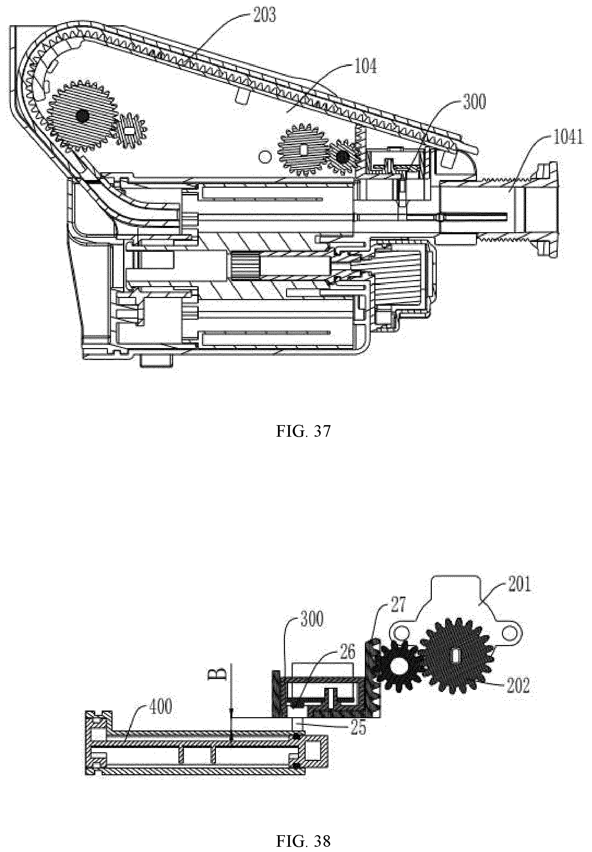

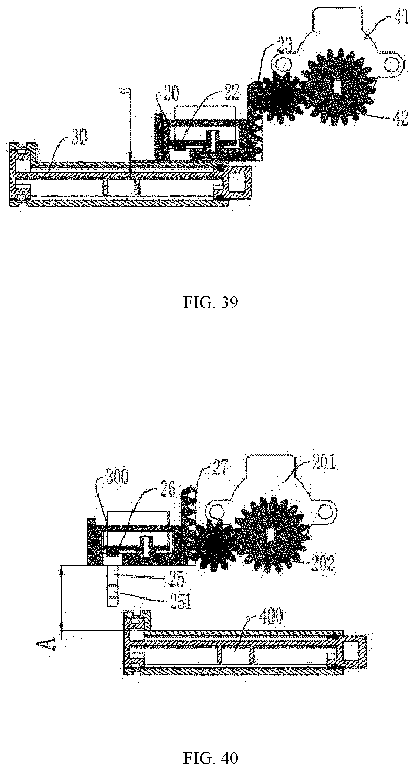

As shown in FIG. 37 to FIG. 42, the test piece for urinalysis includes a base 104, a movable member 400, and a test module. The base 104 is arranged on the toilet, and the movable member 400 and the test module are arranged on the base 104. The movable member 400 can carry a to-be-tested sample, and the test module can move relative to the movable member 400.

In some embodiments, the base 104 may be provided with a through hole 1041, and the through hole 1041 may be in communication with a chamber of the toilet. The chamber of the toilet refers to a space that can accommodate the to-be-tested sample. The movable member 400 can move axially along the through hole 1041, and the movable member 400 passes through the through hole 1041 to extract the to-be-tested sample. The test module can be arranged above the movement path of the movable member 400, and the position relationship between the test module and the movable member 400 can be adjusted according to different states.

As shown in FIG. 37 to FIG. 40, in a specific embodiment, the test module may include a test probe 26 and a test frame, and the test probe 26 is arranged at a bottom of the test frame. The test probe 26 can be used for testing the to-be-tested sample, and the test probe 26 may be of a packaged structure with a sensor, a power source, and an integrated circuit.

In some embodiments, the test module includes two limit members 25, the two limit members 25 are arranged at the bottom of the test frame, the two limit members 25 are arranged on two sides of the test probe 26 separately, and a channel is formed between the two limit members 25. When the test module is in a test state, the movable member 400 can pass through the channel. The channel is provided below the test probe 26 to allow the movable member 400 to pass through in the test state, which can ensure the position relationship between the test probe 26 and the movable member 400, avoids inaccurate test caused by left and right deflection, shaking, etc. of the movable member 400 during moving, and is conducive to improving testing accuracy.

In a specific embodiment, the movable member 400 is provided with limit ribs 431, and the limit member 25 is provided with guide grooves 251 facing the channel. In the test state, the limit ribs 431 are mated with the guide grooves 251, and the movable member 400 moves along the guide grooves 251. Preferably, the limit ribs 431 are arranged on the outer wall of the movable member 400, and the limit ribs 431 are arranged in the moving direction of the movable member 400. The cross-sectional structure of the limit ribs 431 may be semi-circular, elliptical, square, etc. The guide grooves 251 are U-shaped grooves with openings facing the channel, and the cross-sectional structure of the guide grooves 251 may be semi-circular, elliptical, or square.

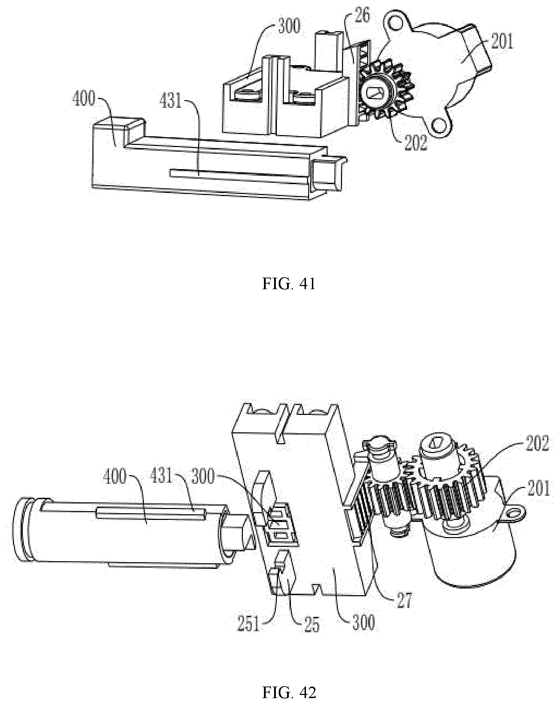

As shown in FIG. 37 and FIG. 41, the movable member 400 has a plane for carrying the to-be-tested sample, and the structure of the movable member 400 may be square or semi-circular.

In some embodiments, the test module may further include a to-be-tested state. In the to-be-tested state, the limit ribs 431 are misaligned with the guide grooves 251, and the limit member 25 is mated with the limit ribs 431 to limit the movement of the movable member 400. Preferably, the limit member 25 is of a plate structure, and the limit member 25 can press against the limit ribs 431 to limit the movement of the movable member 400, which is conducive to ensuring the sealing between the movable member 400 and the base 104. In the to-be-tested state, the sealing relationship between the movable member 400 and the through hole 1041 still needs to be maintained. If the movable member 400 moves into the base 104, the sealing between the movable member 400 and the base 104 may fail.

After the test is completed, the movable member 400 may require replacement of parts or the entire movable member 400 may be replaced. Therefore, the movable member 400 needs to move further into the base 104, and the test module needs to move away from the movable member 400 to make room for the movement. Therefore, the test module includes a yield state, in which the test module moves to a position away from the movable member 400 to yield the movable member.

In a specific embodiment, the distance between the test probe 26 and the movable member 400 is as follows: the distance A between the two in the yield state is longest, followed by the distance B between the two in the to-be-tested state, and the distance C between the two in the test state is shortest, that is, A > B > C.

Understandably, the movement of the test module relative to the movable member 400 may be linear movement or non-linear movement. In a specific embodiment, the test module may move linearly back and forth in a direction perpendicular to the moving direction of the movable member 400. Such a relatively simple movement mode optimizes the movement driving structure of the test module.

As shown in FIG. 37 to FIG. 41, in some embodiments, the test piece for urinalysis further includes a driving module that drives the test module to move. Understandably, the driving module and the test module may be connected by a gear set 202, a belt, or the like. Preferably, the driving module includes a motor 201 and a gear set 202, the motor 201 is mated with the gear set 202, the test module is provided with a guide rail 27, and the guide rail 27 is mated with the gear set 202.

### Thirteenth embodiment:

As shown in FIG. 42, it can be understood that the gear set 202 may include a plurality of gears or one gear.

In this embodiment, an intelligent toilet is further provided. The intelligent toilet includes the aforementioned test piece for urinalysis and a toilet, where the test piece for urinalysis is arranged on the toilet. The test module is arranged on the intelligent toilet, and the test module is movable relative to the movable member 400, which can adjust the distance between the test module and the movable member 400 according to different state requirements, thereby meeting different requirements for accurate test and movement of the movable member 400.

### Fourteenth embodiment:

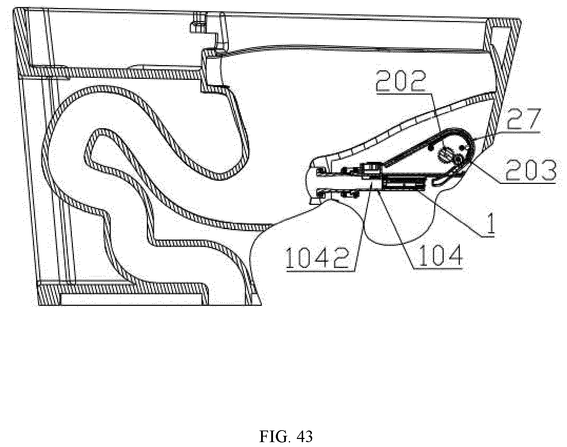

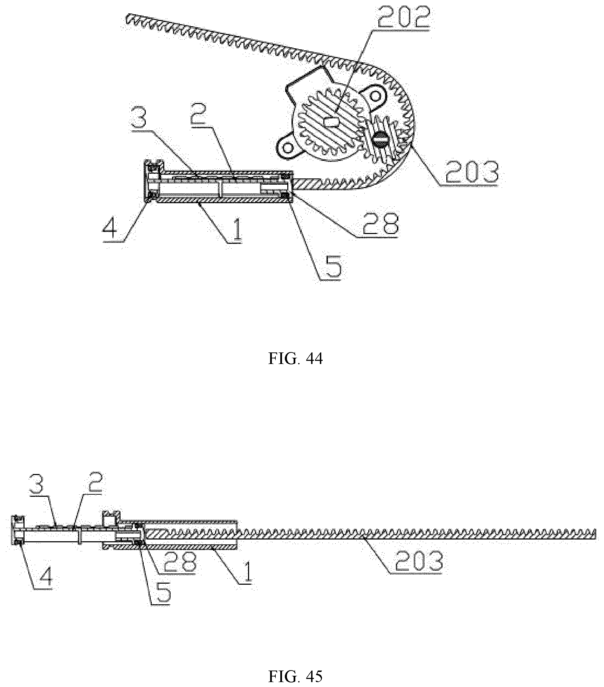

As shown in FIG. 43 and FIG. 44, a transmission mechanism for a test piece for urinalysis includes a driving unit and a test unit; the test unit includes a housing 1 and a test element that extends and retracts relative to the housing 1, the test element includes an isolation portion 28, a piece of test paper 3 is installed on one side of the isolation portion 28, the other side of the isolation portion 28 is mated with the driving unit, then the driving unit drives the test element to move to a preset position for testing, and the isolation portion 28 isolates the test paper 3 from the driving unit.

In this embodiment, the housing 1 is provided with a seal chamber and a second opening, the test element is arranged in the seal chamber, the isolation portion 28 is provided with the second sealing member 5 in sealing fit with the second opening or the inner wall of the seal chamber, and then the test paper 3 is sealed in the seal chamber.

In this embodiment, the housing 1 is further provided with a first opening, the driving unit can drive the test element to extend out of the housing 1 from the first opening and move to an extension position, and the driving unit can also drive the test element to retract to a retraction position.

In this embodiment, the test element is further provided with a first sealing member 4, and the first sealing member 4 can be in sealing fit with the first opening or the inner wall of the seal chamber to seal the test paper 3 in the seal chamber.

In this embodiment, the test paper 3 is located between the first sealing member 4 and the second sealing member 5.

In this embodiment, the test element includes a support 2 and a baffle arranged at a rear end of the support 2, the test paper 3 is installed on the support 2, the baffle forms the isolation portion 28, and the driving unit abuts against the side of the baffle away from the test paper 3.

In this embodiment, the driving unit includes a driving member 203 in transmission fit with the test element, the driving member 203 is a rack, and the driving unit further includes a gear set 202 mated with the rack and a motor driving the gear set 202.

In this embodiment, the base 104 is further provided with a guide rail 27, and the driving member 203 moves along the guide rail 27. Specifically, the base 104 is provided with an accommodating chamber 1042, and the test unit is arranged in the accommodating chamber 1042.

In this embodiment, one end of the driving member 203 abuts against the test element, and the other end of the driving member 203 is bent at a preset angle.

A specific working process of this embodiment is as follows:
The motor drives the gear set 202 to rotate, the gear set 202 meshes with the driving member 203 (rack), and the driving member 203 presses against the isolation portion 28 to push the support 2 to extend to the preset position for testing.

In this solution, the driving unit and the test unit are arranged independently, and the isolation portion 28 isolates the driving unit from the test paper to prevent the driving unit from coming into contact with to-be-tested liquid, so the driving unit does not need to be cleaned, which does not affect the test results, with a simple structure.

This embodiment further provides a toilet for feces examination. The toilet has a toilet bowl and further includes the aforementioned transmission mechanism for the test piece for urinalysis, and the base 104 is installed at the bottom of the toilet bowl.

### Fifteenth embodiment:

As shown in FIG. 45, this embodiment mainly differs from the fourteenth embodiment in that the other end of the driving member 203 in this embodiment extends in a direction away from the test element, and then the driving member 203 is arranged horizontally. The other unspecified parts are consistent with the fourteenth embodiment and will not be repeated here.

### Sixteenth embodiment:

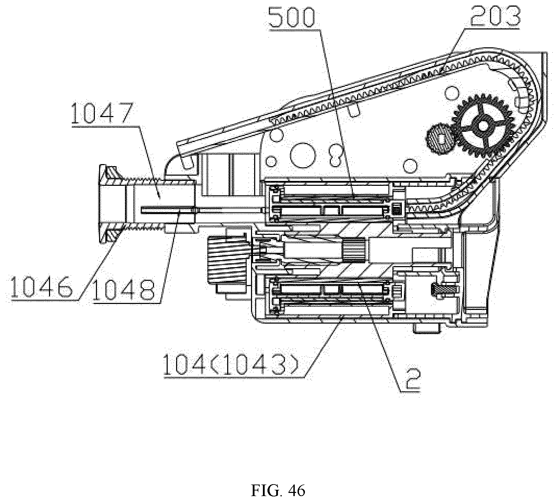

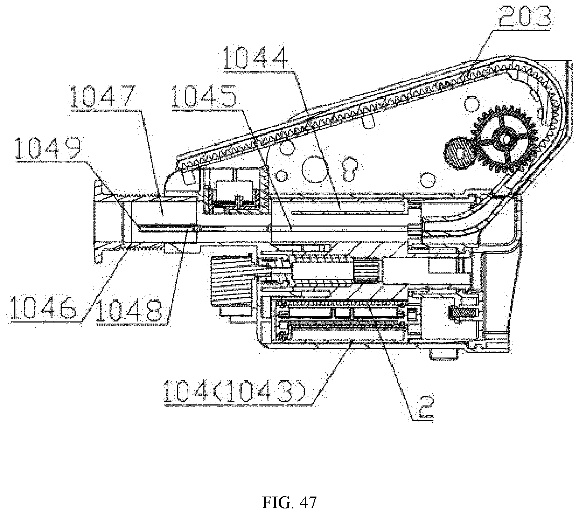

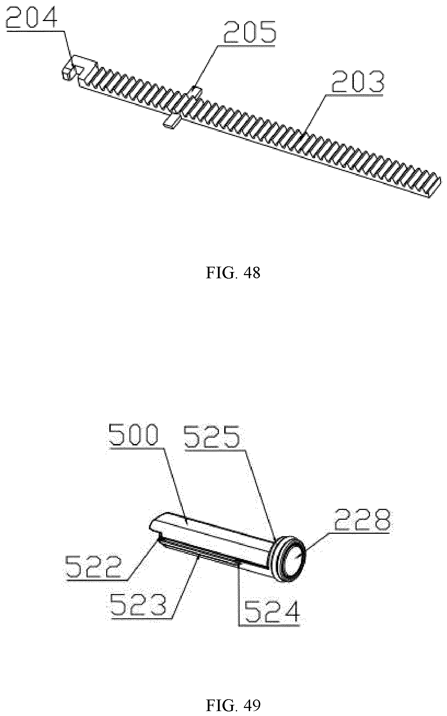

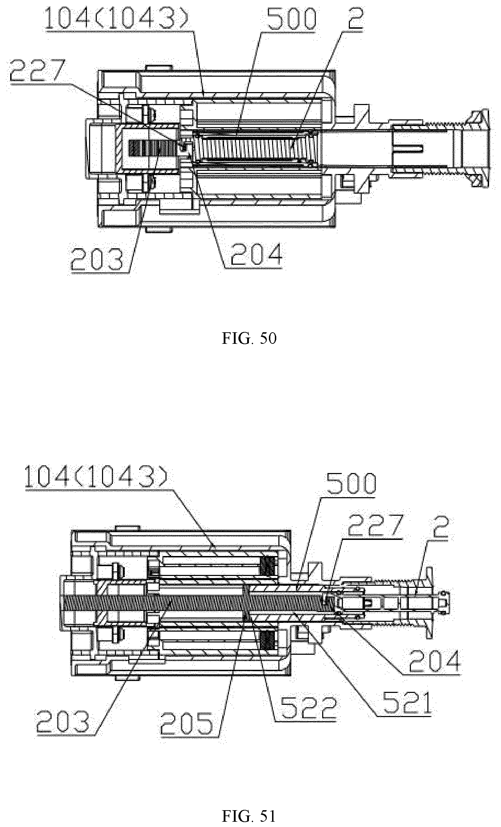

As shown in FIG. 46 to FIG. 52, a segmented transmission mechanism for a test piece for urinalysis includes:
a base 104 having at least one installation portion;
a test box 500 having an accommodating chamber 521, where the test box 500 is movably arranged at the installation portion;
a test element used for testing to-be-tested liquid, where the test element is arranged in the accommodating chamber 521 of the test box 500 in an extensible and retractable manner; and
a driving unit including a driving member, where the driving member is provided with a first driving portion 204 and a second driving portion 205;
where the test element has a first mating portion 227 in transmission fit with the first driving portion 204, and the test box 500 has a second mating portion 522 in transmission fit with the second driving portion 205;
when the test element is in a retraction position, the second driving portion 205 is separated from the second mating portion 522; after the driving member drives the test element to extend a preset distance through the first driving portion 204, the second driving portion 205 abuts against the second mating portion 522 for transmission fit.

In this embodiment, the first driving portion 204 is in clamping fit with the first mating portion 227, and then the driving member drives the test element to extend and retract, that is, the driving member can push the test element outwards or pull the test element inwards. Specifically, a boss 525 is provided on the outer side of the test box 500, and a baffle 228 is correspondingly provided on the outer side of the test element. After the driving member pulls the test element to retract into the test box 500, the baffle 228 abuts against the boss 525, and then the test box 500 is reset through the test element.

In this embodiment, the first driving portion 204 is a hook arranged at the end of the driving member close to the test element, and the first mating portion 227 is a buckle groove arranged at the end of the test element close to the driving member.

In this embodiment, the second driving portion 205 is a convex portion arranged at an angle to the moving direction of the driving member. Specifically, convex portions are provided on two sides of the driving member, and the first driving portions 204 and the second driving portions 205 are spaced apart in the moving direction of the driving member.

In this embodiment, the installation portion is provided with a first guide portion, and the second driving portion 205 can be in guide fit with the first guide portion.

In this embodiment, the test box 500 is correspondingly provided with a second guide portion 523. During the movement of the test box 500 relative to the installation portion, the second guide portion 523 guides and matches the first guide portion. Specifically, the first guide portion is a guide groove, and the second guide portion 523 is a guide rib arranged on the outer wall of the test box 500.

In this embodiment, the base 104 is further provided with a limit portion 1049, and the test box 500 is provided with an abutting portion 524 in limiting fit with the limit portion 1049. When the test box 500 moves to the preset position, the abutting portion 524 abuts against the limit portion 1049 for limiting fit.

In this embodiment, the installation portion includes a movable channel provided by the base 104, and the test box 500 is slid along the movable channel. Specifically, the base 104 includes a sleeve 1043 and a fixed joint 1046, the sleeve 1043 is provided with a plurality of installation chambers 1044, the fixed joint 1046 is provided with a hollow channel 1047, and the installation chambers 1044 are in communication with the hollow channel 1047 to form the installation portion. Specifically, the guide groove includes a first chute 1045 located in the installation chamber 1044 and a second chute 1048 located in the hollow channel 1047, the limit portion 1049 is a limit boss at a tail end of the second chute 1048, and the abutting portion 524 is a tail end of a guide rib.

In this embodiment, the test element includes a support and test paper placed on the support.

In this embodiment, the driving member is a rack, the driving unit further includes a motor and a gear set, and the motor drives the rack through the gear set.

A specific working process of this embodiment is as follows:
In the initial state, the test element is in the retraction position, and the second driving portion 205 is separated from the second mating portion 522 of the test box 500. After the driving member drives the test element to extend a preset distance through the first driving portion 204, the second driving portion 205 abuts against the second mating portion 522 for transmission fit to drive the test element and the test box 500 to move forward together until the abutting portion 524 of the test box 500 abuts against the limit portion 1049 of the installation portion for limiting, the test box 500 and the test element stop moving, and the test element extends from the movable channel into the toilet bowl.

When retracting, the driving member pulls the test element to retract into the test box 500, the baffle 228 abuts against the boss 525, and then the test box 500 is reset through the test element.

In this solution, one driving member drives the test element and the test box 500 to move sequentially, with a simple structure and cost saving.

### Seventeenth embodiment:

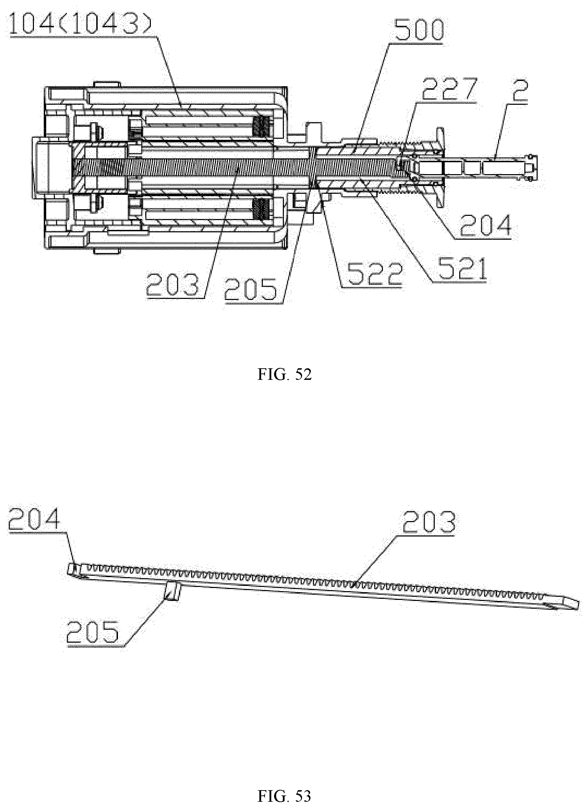

As shown in FIG. 53, this embodiment mainly differs from the sixteenth embodiment in that the second driving portion 205 is a convex portion arranged on a bottom wall of the driving member. The other unspecified parts are consistent with the sixteenth embodiment and will not be repeated here.

Finally, it should be noted that the above embodiments are only for explaining, but not limiting, the technical solutions of the present invention; although the present invention is described in detail with reference to the foregoing embodiments, those of ordinary skill in the art should understood that the technical solutions described in the foregoing embodiments can be modified, or some of the technical features can be equivalently substituted; and these modifications or substitutions do not make the essence of the corresponding technical solutions depart from the spirit and scope of the technical solutions of the present invention.

## Claims

1. A test piece for urinalysis, installed on a toilet seat for human urinalysis, comprising a housing, a support, and test paper, wherein the support is movably arranged on the housing, and the test paper is placed on the support to move with the support relative to the housing; in an initial state, the test paper is sealed in the housing; during testing, the test paper is exposed out of the housing by driving the support.

2. The test piece for urinalysis according to claim 1, wherein the support is arranged on the housing in an extensible and retractable manner, the housing is provided with a first opening for the support to extend and retract relative to the housing, and the support is movable between an extension position relative to the housing and a retraction position relative to the housing; in the initial state, the support is in the retraction position, and a seal chamber for sealing the test paper is formed between the support and the housing, or both the support and the test paper are arranged in a seal chamber formed by an inner cavity of the housing; during testing, the support is located at the extension position, and the test paper is exposed out of the seal chamber from the first opening.

3. The test piece for urinalysis according to claim 2, wherein a first sealing member is provided between the support and the housing; when the support is in the retraction position, the first opening is sealed between the support and the housing by the first sealing member to form the seal chamber for accommodating the test paper, or the first sealing member is in sealing fit with an inner wall of the housing to form the seal chamber for accommodating the test paper.

4. The test piece for urinalysis according to claim 2, wherein the first opening is sealed by a penetrable first sealing film to form the seal chamber by the inner cavity of the housing, and the support and the test paper are sealed by the first sealing film in the seal chamber formed by the inner cavity of the housing.

5. The test piece for urinalysis according to claim 2, wherein the housing is further provided with a second opening for driving the support; a second sealing member is provided between the support and the housing; when the support is in the retraction position, the second opening is sealed between the support and the housing by the second sealing member to form the seal chamber for accommodating the test paper; or the second opening is sealed by a penetrable second sealing film to form the seal chamber by the inner cavity of the housing, and the support and the test paper are sealed by the second sealing film in the seal chamber formed by the inner cavity of the housing.

6. The test piece for urinalysis according to claim 5, wherein the first opening is opposite to the second opening, a driving portion is provided at the end of the support close to the second opening, and the driving portion is located outside the housing when the support is in the retraction position.

7. The test piece for urinalysis according to claim 1, wherein an elastic member is provided between the housing and the support, and the support extends to the extension position by overcoming elastic force of the elastic member under the action of external driving force; when the external driving force applied to the support is removed, the support is reset to the retraction position under the elastic force of the elastic member.

8. The test piece for urinalysis according to claim 1, further comprising a first magnetic member arranged on the support, wherein the first magnetic member is used for cooperating with a magnetic driving component, and the support is driven by magnetic force formed between the magnetic driving component and the first magnetic member; the magnetic driving component is a second magnetic member or electromagnet, and there is an attractive magnetic force or repellent magnetic force between the first magnetic member and the second magnetic member or electromagnet.

9. The test piece for urinalysis according to claim 1, wherein at least the portion of the side wall of the housing corresponding to the test paper is made of a transparent material.

10. The test piece for urinalysis according to claim 9, wherein at least the portion of the side wall of the housing corresponding to the test paper is a straight wall.

11. The test piece for urinalysis according to claim 1, wherein the cross-sectional shape of the outer surface of the portion of the side wall of the housing that matches a test hole on the toilet seat matches the cross-sectional shape of the test hole.

12. The test piece for urinalysis according to claim 1, further comprising a test unit and a driving unit, wherein the test unit comprises the housing and a test element that extends and retracts relative to the housing, and the test element comprises the support and the test paper; the housing is provided with the first opening, the driving unit can drive the test element to extend out of the housing from the first opening and move to the extension position, and the driving unit can also drive the test element to retract to the retraction position.

13. The test piece for urinalysis according to claim 12, wherein the housing is a hollow sleeve; the housing is further provided with the second opening, and the test element extends from the second opening to connect with the driving unit, or the driving unit extends from the second opening into the housing to connect with the test element.

14. The test piece for urinalysis according to claim 12, wherein the driving unit comprises a driving member in transmission fit with the test unit and a power source that provides driving force to the driving member; the driving member comprises a gear and a rack, or the driving member comprises a transmission belt; the power source is a water pressure source, a pneumatic pressure source, a magnetic component, or a motor.

15. The test piece for urinalysis according to claim 1, wherein the housing has the seal chamber and the first opening; the support is arranged in the seal chamber and extends and retracts relative to the housing through the first opening, the first sealing member is provided at a front end of the support, and the first sealing member is in sealing fit with the first opening when the support moves to the retraction position; the upper surface of the support is a mating surface for smooth transition, a push portion close to the mating surface is provided at the first opening, and the push portion can push a foreign matter on the mating surface during the retraction of the support.

16. The test piece for urinalysis according to claim 15, wherein an upper wall of the seal chamber is parallel and close to the mating surface; the mating surface is a straight or curved surface, and the seal chamber is cylindrical with a straight or curved upper wall; an upper wall of the first opening forms the push portion.

17. The test piece for urinalysis according to claim 15, wherein the support is provided with a sink, a convex portion higher than the sink is provided at the front end of the support, and the first sealing member is arranged on the convex portion; the test paper is laid on the sink; the test piece for urinalysis further comprises a gland, which presses the test paper onto the support; an upper surface of the gland and an upper surface of the convex portion have the same height and are mated to form the mating surface.

18. The test piece for urinalysis according to claim 1, further comprising a gland, wherein the support has a limit portion; the test paper is provided with a plurality of test paper blocks, and the test paper blocks are used for contact reaction with to-be-tested liquid; the gland has a pouring area and a plurality of isolation ribs, the isolation ribs divide the pouring area into a plurality of isolation chambers, each of the isolation chambers corresponds to each of the test paper blocks, the isolation ribs isolate the adjacent test paper blocks, the gland is further provided with a clamping portion in clamping fit with the limit portion, and the gland is in clamping fit with the support to confine the test paper between the gland and the support.

19. The test piece for urinalysis according to claim 18, wherein the clamping portion comprises a plurality of buckles spaced apart in a length direction of the gland, and the limit portion comprises convex ribs extending in a length direction of the support; the buckles are arranged at the positions corresponding to the isolation chambers; the test paper blocks are color blocks corresponding to different human health indicators.

20. The test piece for urinalysis according to claim 18, wherein the side of the isolation chamber adjacent to the outside of the pouring area has a drainage notch, and then the to-be-tested liquid can overflow from the drainage notch to the outside; the side of the isolation chamber adjacent to the outside of the pouring area is an outer wall, an inner wall of the isolation chamber and a front wall and a rear wall of the isolation chamber are all higher than the outer wall, and then an upper area of the outer wall forms the drainage notch; the inner wall of the isolation chamber and the front wall and rear wall of the isolation chamber are all higher than the test paper block; the shape and size of the isolation chambers match the test paper blocks.

21. The test piece for urinalysis according to claim 18, wherein the isolation ribs comprise a plurality of transverse ribs arranged in a width direction of the gland and longitudinal ribs arranged in the length direction of the gland, and the transverse ribs intersect and match the longitudinal ribs to separate the plurality of isolation chambers.

22. The test piece for urinalysis according to claim 1, further comprising a test unit and a driving unit, wherein the test unit comprises the housing and a test element that extends and retracts relative to the housing; the test element comprises the support and the test paper, as well as a baffle arranged at a rear end of the support; the test paper is installed on the support, the baffle forms an isolation portion, and the driving unit abuts against the side of the baffle away from the test paper.

23. The test piece for urinalysis according to claim 22, wherein the driving unit comprises a driving member in transmission fit with the test element; one end of the driving member abuts against the test element, and the other end of the driving member is bent at a preset angle, or the other end of the driving member extends in a direction away from the test element.

24. The test piece for urinalysis according to claim 1, further comprising a test unit and a driving unit, wherein the test unit comprises the housing and a test element that extends and retracts relative to the housing; the test element comprises the support and the test paper; the driving unit comprises a driving member, and the driving member is provided with a first driving portion and a second driving portion; the test element has a first mating portion in transmission fit with the first driving portion, and a test box has a second mating portion in transmission fit with the second driving portion; when the test element is in the retraction position, the second driving portion is separated from the second mating portion; after the driving member drives the test element to extend a preset distance through the first driving portion, the second driving portion abuts against the second mating portion for transmission fit.

25. A test piece for urinalysis, comprising:
a support having a limit portion;
test paper provided with a plurality of test paper blocks, wherein the test paper blocks are used for contact reaction with to-be-tested liquid; and
a gland having a pouring area and a plurality of isolation ribs, wherein the isolation ribs divide the pouring area into a plurality of isolation chambers, each of the isolation chambers corresponds to each of the test paper blocks, the isolation ribs isolate the adjacent test paper blocks, the gland is further provided with a clamping portion in clamping fit with the limit portion, and the gland is in clamping fit with the support to confine the test paper between the gland and the support.

26. The test piece for urinalysis according to claim 25, wherein the clamping portion comprises a plurality of buckles spaced apart in a length direction of the gland, and the limit portion comprises convex ribs extending in a length direction of the support; the buckles are arranged at the positions corresponding to the isolation chambers; the test paper blocks are color blocks corresponding to different human health indicators.

27. The test piece for urinalysis according to claim 25, wherein the side of the isolation chamber adjacent to the outside of the pouring area has a drainage notch, and then the to-be-tested liquid can overflow from the drainage notch to the outside; the side of the isolation chamber adjacent to the outside of the pouring area is an outer wall, an inner wall of the isolation chamber and a front wall and a rear wall of the isolation chamber are all higher than the outer wall, and then an upper area of the outer wall forms the drainage notch; the inner wall of the isolation chamber and the front wall and rear wall of the isolation chamber are all higher than the test paper block; the shape and size of the isolation chambers match the test paper blocks.

28. The test piece for urinalysis according to claim 25, wherein the isolation ribs comprise a plurality of transverse ribs arranged in a width direction of the gland and longitudinal ribs arranged in the length direction of the gland, and the transverse ribs intersect and match the longitudinal ribs to separate the plurality of isolation chambers.

29. The test piece for urinalysis according to claim 25, wherein the isolation chambers penetrate an upper surface and a lower surface of the gland; one of the support and the gland is provided with fool-proof ribs, the other of the support and the gland is provided with fool-proof clamping grooves, and the fool-proof ribs are mated with the fool-proof clamping grooves.

30. A test piece for urinalysis, comprising a test module, an identification module, and a driving unit, wherein the test module comprises a support and test paper arranged on the support, and the test paper is provided with a plurality of test paper blocks; the identification module is configured to collect data from the test paper blocks, and the identification module comprises at least one sensor and at least one light source; the identification module simultaneously tests at least two of the test paper blocks, the identification module further comprises a light blocking plate, the light blocking plate is provided with an emitting hole corresponding to the light source and a receiving hole corresponding to the sensor, and the driving unit is in transmission fit with the test module and/or the identification module.

31. The test piece for urinalysis according to claim 30, wherein the identification module comprises two sensors and one light source, and the light source is located between the two sensors; or the identification module comprises one sensor and two light sources, and the sensor is located between the two light sources; or the quantity of light sources is the same as that of sensors, each of the light sources corresponds to each of the sensors, and the light sources and the sensors are arranged in left and right directions of the test paper blocks, or the light sources and the sensors are arranged in front and rear directions of the test paper blocks.

32. The test piece for urinalysis according to claim 30, wherein the test paper blocks are arranged in two rows in a length direction of the support, and the two sensors correspond one to one with two of the test paper blocks in the width direction of the support; the driving unit and the test module and/or the identification module move in the length direction of the support, and the spacing between the test paper blocks arranged in the length direction of the support is equal.

33. The test piece for urinalysis according to claim 30, wherein the quantity of emitting holes is the same as that of light sources, and the emitting holes correspond one to one with the light sources; the quantity of receiving holes is the same as that of sensors, and the receiving holes correspond one to one with the sensors; both the emitting holes and the receiving holes are sealed by transparent members.

34. The test piece for urinalysis according to claim 30, wherein the sensor is a color sensor, the light source is an LED light, the test piece for urinalysis further comprises a circuit board, and the color sensor and the LED light are both arranged on the circuit board; the driving unit comprises a rack in transmission fit with the support, the test piece for urinalysis further comprises a motor and a gear set, and the motor drives the rack to move through the gear set.

35. A test piece for urinalysis, arranged on a toilet, comprising:
a base arranged on the toilet;
a movable member capable of carrying a to-be-tested sample and movable along the base; and
a test module arranged on the base, wherein the test module is movable relative to the movable member, and the test module comprises a test state and a yield state;
wherein in the test state, the test module is close to the movable member, the movable member carries the to-be-tested sample to a position corresponding to the test module, and the test module tests the to-be-tested sample; in the yield state, the test module moves to a position away from the movable member to yield the movable member.

36. The test piece for urinalysis according to claim 35, wherein the test module further comprises a to-be-tested state, and in the to-be-tested state, the test module limits the movement of the movable member; specifically, the test module further comprises a limit member, the movable member is provided with limit ribs, and the limit member is provided with guide grooves; in the test state, the limit ribs are correspondingly mated with the guide grooves, and the movable member moves along the guide grooves; in the to-be-tested state, the limit ribs are misaligned with the guide grooves, and the limit member is mated with the limit ribs to limit the movement of the movable member.

37. The test piece for urinalysis according to claim 36, wherein the test module comprises a test frame and a test probe, the test probe is arranged at a bottom of the test frame, two limit members are arranged on two sides of the test frame separately, and a channel is provided between the two limit members; in the test state, the movable member can pass through the channel.

38. The test piece for urinalysis according to claim 35, wherein the base is provided with a through hole, the movable member moves axially along the through hole, and the movable member passes through the through hole to extract the to-be-tested sample; the test module moves linearly back and forth in a direction perpendicular to the direction of movement of the movable member.

39. The test piece for urinalysis according to claim 35, further comprising a driving module that drives the test module to move, wherein the driving module comprises a motor and a gear set, the motor is mated with the gear set, a guide rail is provided on the test module, and the guide rail is mated with the gear set.

40. A transmission mechanism for a test piece for urinalysis, comprising a driving unit and a test unit, wherein the test unit comprises a housing and a test element that extends and retracts relative to the housing, the test element comprises an isolation portion, a piece of test paper is installed on one side of the isolation portion, the other side of the isolation portion is mated with the driving unit, then the driving unit drives the test element to move to a preset position for testing, and the isolation portion isolates the test paper from the driving unit.

41. The transmission mechanism for the test piece for urinalysis according to claim 40, wherein the housing is provided with a seal chamber and a second opening, the test element is arranged in the seal chamber, the isolation portion is provided with a second sealing member in sealing fit with the second opening or an inner wall of the seal chamber, and then the test paper is sealed in the seal chamber.

42. The transmission mechanism for the test piece for urinalysis according to claim 41, wherein the housing is provided with a first opening, the driving unit can drive the test element to extend out of the housing from the first opening to move to an extension position, and the driving unit can also drive the test element to retract to a retraction position; the test element is further provided with a first sealing member, the first sealing member can be in sealing fit with the first opening or the inner wall of the seal chamber, and then the test paper is sealed in the seal chamber.

43. The transmission mechanism for the test piece for urinalysis according to claim 42, wherein the test paper is located between the first sealing member and the second sealing member.

44. The transmission mechanism for the test piece for urinalysis according to claim 40, wherein the test element comprises a support and a baffle arranged at a rear end of the support, the test paper is installed on the support, the baffle forms the isolation portion, and the driving unit abuts against the side of the baffle away from the test paper.

45. The transmission mechanism for the test piece for urinalysis according to claim 40, wherein the driving unit comprises a driving member in transmission fit with the test element, the driving member is a rack, and the driving unit further comprises a gear set mated with the rack and a motor driving the gear set; the transmission mechanism further comprises a base, the base is further provided with a guide rail, and the driving member moves along the guide rail.

46. The transmission mechanism for the test piece for urinalysis according to claim 45, wherein one end of the driving member abuts against the test element, and the other end of the driving member is bent at a preset angle, or the other end of the driving member extends in a direction away from the test element.

47. A segmented transmission mechanism for a test piece for urinalysis, comprising:
a base having at least one installation portion;
a test box having an accommodating chamber, wherein the test box is movably arranged at the installation portion;
a test element used for testing to-be-tested liquid, wherein the test element is arranged in the accommodating chamber of the test box in an extensible and retractable manner; and
a driving unit comprising a driving member, wherein the driving member is provided with a first driving portion and a second driving portion;
wherein the test element has a first mating portion in transmission fit with the first driving portion, and the test box has a second mating portion in transmission fit with the second driving portion;
when the test element is in a retraction position, the second driving portion is separated from the second mating portion; after the driving member drives the test element to extend a preset distance through the first driving portion, the second driving portion abuts against the second mating portion for transmission fit.

48. The segmented transmission mechanism for the test piece for urinalysis according to claim 47, wherein the first driving portion is engaged with the first mating portion, and then the driving member drives the test element to extend and retract; specifically, the first driving portion is a hook arranged at the end of the driving member close to the test element, and the first mating portion is a buckle groove arranged at the end of the test element close to the driving member.

49. The segmented transmission mechanism for the test piece for urinalysis according to claim 47, wherein the second driving portion is a convex portion arranged at an angle to the direction of movement of the driving member, and the first driving portion and the second driving portion are spaced apart in the direction of movement of the driving member; in addition, the installation portion is provided with a first guide portion, and the second driving portion can guide and mate with the first guide portion; the test box is correspondingly provided with a second guide portion, and the second guide portion is mated with the first guide portion during the movement of the test box relative to the installation portion.

50. The segmented transmission mechanism for the test piece for urinalysis according to claim 47, wherein the base is further provided with a limit portion, and the test box is provided with an abutting portion in limiting fit with the limit portion; when the test box moves to a preset position, the abutting portion abuts against the limit portion for limiting fit; the installation portion comprises a movable channel provided in the base, and the test box is slid along the movable channel.

51. The segmented transmission mechanism for the test piece for urinalysis according to claim 47, wherein the test element comprises a support and test paper placed on the support; the driving member is a rack, the driving unit further comprises a motor and a gear set, and the motor drives the rack through the gear set.

52. A toilet, comprising a toilet seat provided with a test hole, and further comprising the test piece for urinalysis according to any one of claims 1 to 24, wherein the housing of the test piece for urinalysis is detachably or movably arranged at the test hole.

53. The toilet according to claim 52, wherein a casing is fixedly installed at the test hole, and the housing of the test piece for urinalysis is detachably or movably installed inside the casing; the test hole is arranged at a bottom of a toilet bowl; the bottom of the toilet bowl is further provided with an installation hole, a nozzle assembly is installed inside the installation hole, and the nozzle assembly sprays water to the bottom of the toilet bowl.

54. The toilet according to claim 52, wherein there is a spacing gap between an upper surface of the housing and the test hole, a blocking surface extending upwards is provided at the front end of the housing, and the blocking surface is used for filling the spacing gap.

55. The toilet according to claim 54, wherein the blocking surface is located on the same plane as the push portion at the first opening of the housing, or the blocking surface is misaligned with the push portion.

56. The toilet according to claim 54, wherein a third sealing member in sealing fit with the test hole is provided on the housing close to the blocking surface.

57. A toilet, comprising the test piece for urinalysis according to any one of claims 1 to 32; or comprising the transmission mechanism for the test piece for urinalysis according to any one of claims 33 to 39; or comprising the segmented transmission mechanism for the test piece for urinalysis according to any one of claims 40 to 44.
